(19) Europäisches Patentamt
European Patent Office
Office européen des brevets

(11) EP 4 653 415 A1

(12) EUROPEAN PATENT APPLICATION
published in accordance with Art. 153(4) EPC

(43) Date of publication:
**26.11.2025 Bulletin 2025/48**

(21) Application number: **24744736.0**

(22) Date of filing: **19.01.2024**

(51) International Patent Classification (IPC):
***C07C 37/84** (2006.01)* ***C07C 37/52** (2006.01)*
***C07C 39/16** (2006.01)* ***C08G 64/20** (2006.01)*

(52) Cooperative Patent Classification (CPC):
**C07C 37/52; C07C 37/84; C07C 39/16; C08G 64/20**

(86) International application number:
**PCT/JP2024/001424**

(87) International publication number:
**WO 2024/154816 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.01.2023 JP 2023007436**

(71) Applicant: **Mitsubishi Chemical Corporation**
**Tokyo 100-8251 (JP)**

(72) Inventors:
- **NAKASHIMA, Yukie**
  **Tokyo 100-8251 (JP)**
- **KINOSHITA UMENO, Misaki**
  **Tokyo 100-8251 (JP)**
- **NAGANO, Fumika**
  **Tokyo 100-8251 (JP)**

(74) Representative: **Vossius & Partner Patentanwälte Rechtsanwälte mbB**
**Siebertstrasse 3**
**81675 München (DE)**

# (54) METHOD FOR PRODUCING BISPHENOL AND METHOD FOR PRODUCING RECYCLED POLYCARBONATE RESIN

(57) Provided is a method for producing a bisphenol, by which it is possible to obtain a high-purity bisphenol not containing another rein derived from a coating layer, an alloy material, and the like. The method for producing a bisphenol is a method for producing a bisphenol by removing, from a mixed solution containing a bisphenol and another resin other than a polycarbonate resin, the another resin to obtain the bisphenol, the method including a step 1 in which the bisphenol is precipitated out of an aromatic monoalcohol-containing solution in which the bisphenol and the another resin are dissolved in a solvent containing an aromatic monoalcohol to obtain a slurry of the bisphenol, and the slurry of the bisphenol is then subjected to solid-liquid separation to obtain a crude cake of the bisphenol and a mother liquor.

EP 4 653 415 A1

## Description

### Technical Field

The present invention relates to a method for producing a bisphenol. The present invention also relates to a method for producing a recycled polycarbonate resin by using the bisphenol as a raw material.

### Background Art

Plastics are absolutely necessary for modern society for their excellent functions and properties and are produced and consumed in large amounts not only in Japan but also across the world. For sustainable development, recycling of plastic resources after consumption is important, and there are roughly three methods therefor: material recycling that is a method of recycling them into raw materials for plastic products; chemical recycling that is a method of recycling them into chemical raw materials; and thermal recycling that is a method of using them as energy source.

In Japan, thermal recycling is most commonly used in terms of the waste plastic recycling rates of respective recycling laws. The amount of heat generated by combusting plastics is comparable to that generated by combusting coal or petroleum, and therefore thermal energy obtained by combusting plastics is effectively used for electric power generation etc. However, in Europe and the United States, thermal recycling is often not regarded as recycling, and therefore material recycling and chemical recycling are required to be further promoted. Even polycarbonate resins, which are used in various fields for their transparency, mechanical properties, flame retardance, dimensional stability, and electric characteristics, are no exception.

Polycarbonate resins are excellent in processability and durability and also have high transparency. Therefore, such polycarbonate resins are used not only for housings of home electrical appliances and optical recording discs (e.g., CDs) but also for car head lamp lenses, carport roof materials, highway noise barriers, and the like which are used outdoors for a long time. Most of these polycarbonate resin products for outdoor use are coated with an acrylic resin or the like to improve scratch resistance of their surfaces.

Further, many alloy materials are distributed which are formed by compounding a polycarbonate resin with a resin other than the polycarbonate resin, such as a polyester resin or an ABS resin, (hereinafter sometimes simply referred to as "another resin") to achieve higher performance.

Many methods for material recycling or chemical recycling of a polycarbonate resin have been reported. As material recycling methods, there are a known method in which the metal surface coatings of optical recording discs are removed by treatment with an aqueous alkali solution to recover a polycarbonate resin used as a base material (Patent Literature 1) and a known method in which optical recording discs are dissolved in a solvent and then only a polycarbonate resin is recovered by precipitation using a mixed solution of water and an alcohol (Patent Literature 2).

As chemical recycling methods, there are a known method in which a waste polycarbonate resin is hydrolyzed to recover a polycarbonate resin as an aqueous alkali solution containing a bisphenol sodium salt (Patent Literature 3), a known method in which optical recording discs are subjected to alcoholysis to recover a bisphenol and a dialkyl carbonate (Patent Literature 4), and a known method in which a waste polycarbonate resin is subjected to phenolysis to recover a bisphenol and diphenyl carbonate (Patent Literature 5).

### Citation List

#### Patent Literatures

Patent Literature 1: Japanese Patent Laid-Open No. 2001-310970
Patent Literature 2: Japanese Patent Laid-Open No. 2009-84538
Patent Literature 3: Japanese Patent Laid-Open No. 2005-179460
Patent Literature 4: Japanese Patent Laid-Open No. 2001-160243
Patent Literature 5: Japanese Patent Laid-Open No. 7-207059


### Summary of Invention

#### Technical Problem

A general method for recycling a polycarbonate resin product is material recycling, but material recycling has a drawback that the uses of a recycled product are limited. For example, a polycarbonate resin product having a coating layer

of an acrylic resin or the like is difficult to recycle for building materials or car head lamp lenses required to have transparency because when such a polycarbonate resin product is subjected to material recycling, silver streaks are caused during injection molding. Therefore, most of polycarbonate resin products to be subjected to material recycling are colored so as to be recycled as opaque materials.

Further, in the case of material recycling, it is impossible to separate a colorant or another resin from a polycarbonate resin. Therefore, the only way to obtain a resin product having desired quality and performance is to set the mixing ratio of a waste polycarbonate resin at a low level, which is however not efficient from the viewpoint of cyclical use of resources. For these reasons, a waste polycarbonate resin containing a colorant or another resin is preferably purified and recovered as a monomer by chemical recycling.

However, as a result of an attempt to obtain a bisphenol through depolymerization of a waste polycarbonate resin containing another resin, it has become obvious that the obtained bisphenol is contaminated with a polymer. Also, it has been found that the bisphenol containing the another resin is difficult to dry because a solvent is less likely to evaporate.

Such contamination with a polymer is not preferred because the purity of the bisphenol is, of course, reduced and there is also a possibility that an unintended side reaction, coloration, operation trouble due to gas generation, etc. occur when producing a recycled polycarbonate resin by using the bisphenol as a raw material. For this reason, there has been a demand for a method for purifying a bisphenol by efficiently separating and removing, from the bisphenol, a polymer contained in a waste polycarbonate resin.

The present invention has been made in light of the above circumstances, and it is an object of the present invention to provide a method for producing a bisphenol, by which it is possible to obtain a high-purity bisphenol not containing another resin derived from a coating layer, an alloy material, and the like. It is also an object to provide a method for producing a recycled polycarbonate resin by using the bisphenol as a raw material.

Solution to Problem

The present inventor has intensively studied to achieve the above objects and, as a result, has found that the above objects can be achieved by the following invention, and this finding has led to the completion of the present invention. Specifically, the present invention relates to the following aspects.

<1> A method for producing a bisphenol by removing, from a mixed solution containing a bisphenol and a resin other than a polycarbonate resin (hereinafter referred to as "another resin"), the another resin to obtain the bisphenol, the method including a step 1 in which the bisphenol is precipitated out of an aromatic monoalcohol-containing solution in which the bisphenol and the another resin are dissolved in a solvent containing an aromatic monoalcohol to obtain a slurry of the bisphenol, and the slurry of the bisphenol is then subjected to solid-liquid separation to obtain a crude cake of the bisphenol and a mother liquor.

<2> The method for producing a bisphenol according to the above <1>, wherein before the bisphenol is precipitated, the mixed solution or the aromatic monoalcohol-containing solution is subjected to solid-liquid separation to obtain a homogeneous solution.

<3> The method for producing a bisphenol according to the above <1> or <2>, including, before the step 1, a depolymerization step in which a polycarbonate resin contained in a polycarbonate resin complex containing the polycarbonate resin and the another resin is subjected to depolymerization to obtain a decomposed liquid containing the bisphenol and the another resin, wherein the mixed solution corresponds to the decomposed liquid.

<4> The method for producing a bisphenol according to the above <3>, wherein the mixed solution is one obtained by performing the depolymerization in the presence of the aromatic monoalcohol in the depolymerization step, and the aromatic monoalcohol-containing solution corresponds to the mixed solution.

<5> The method for producing a bisphenol according to the above <3>, wherein the mixed solution is one obtained by performing the depolymerization in the absence of the aromatic monoalcohol in the depolymerization step, and the aromatic monoalcohol-containing solution is one obtained by replacing a solvent of the obtained mixed solution with the aromatic monoalcohol.

<6> The method for producing a bisphenol according to any one of the above <3> to <5>, wherein the polycarbonate resin complex is at least one selected from the group consisting of the following (c1) to (c8):

(c1) a molded body of a polymer alloy of the polycarbonate resin and the another resin;
(c2) a resin molded body of the polycarbonate resin, the resin molded body having a surface coated with the another resin;
(c3) a resin molded body of the polycarbonate resin, the resin molded body having a surface coated with a polymer alloy of the polycarbonate resin and the another resin;
(c4) a resin molded body of a polymer alloy of the polycarbonate resin and the another resin, the resin molded body having a surface coated with the another resin;

(c5) a resin molded body of a polymer alloy of the polycarbonate resin and the another resin, the resin molded body having a surface coated with a polymer alloy of the polycarbonate resin and the another resin;
(c6) a resin molded body of a polymer alloy of the polycarbonate resin and the another resin, the resin molded body having a surface coated with the polycarbonate resin;
(c7) a resin molded body of the another resin, the resin molded body having a surface coated with the polycarbonate resin; and
(c8) a resin molded body of the another resin, the resin molded body having a surface coated with a polymer alloy of the polycarbonate resin and the another resin.

<7> The method for producing a bisphenol according to any one of the above <1> to <6>, wherein the aromatic monoalcohol contains phenol or cresol.
<8> The method for producing a bisphenol according to any one of the above <1> to <7>, wherein the aromatic monoalcohol is phenol, and, in the step 1, the bisphenol is precipitated as a crystalline adduct of the bisphenol and phenol to obtain a crude cake of the crystalline adduct of the bisphenol and phenol.
<9> The method for producing a bisphenol according to any one of the above <1> to <8>, wherein an amount-of-substance ratio of the aromatic monoalcohol to the bisphenol contained in the aromatic monoalcohol-containing solution, out of which the bisphenol is to be precipitated, is 1.0 or more (for example, 1.0 or more and 10 or less).
<10> The method for producing a bisphenol according to any one of the above <1> to <9>, including, after the step 1, a step 2 in which a washing liquid is supplied to the crude cake of the bisphenol to wash the crude cake of the bisphenol, so that a purified cake of the bisphenol is obtained.
<11> The method for producing a bisphenol according to the above <10>, wherein the washing liquid is at least one selected from the group consisting of an aromatic monoalcohol, an aliphatic monoalcohol, a ketone, an aromatic hydrocarbon, an aliphatic hydrocarbon, and water.
<12> The method for producing a bisphenol according to any one of the above <1> to <11>, including a step in which the crude cake of the bisphenol or a purified cake obtained by washing the crude cake is dissolved in a solvent or melted to obtain a bisphenol solution, and the bisphenol solution is then brought into contact with a solid adsorbent to obtain an adsorption-purified liquid.
<13> The method for producing a bisphenol according to any one of the above <1> to <12>, wherein the another resin is at least one selected from the group consisting of an acrylic resin, polyethylene terephthalate, polybutylene terephthalate, an ABS resin, a polyamide, a phenol resin, a polyurethane, polylactic acid, and a silicone resin.
<14> The method for producing a bisphenol according to any one of the above <1> to <13>, wherein the bisphenol is 2,2-bis(4-hydroxyphenyl)propane.
<15> A method for producing a recycled polycarbonate resin, the method including the steps of: obtaining a bisphenol by the method for producing a bisphenol according to any one of the above <1> to <14>; and producing a recycled polycarbonate resin by using a bisphenol raw material containing the obtained bisphenol.

<X1> The method for producing a bisphenol according to the above <11>, wherein the washing liquid is at least one selected from the group consisting of phenol, cresol, methanol, ethanol, acetone, cyclohexanone, benzene, toluene, xylene, hexane, heptane, cyclohexane, and water.
<X2> The method for producing a bisphenol according to the above <12>, including: a water washing step in which the adsorption-purified liquid is mixed with water, the resultant is then separated into an organic phase containing the bisphenol and a water phase by phase separation, and the water phase is removed to obtain the organic phase; and a purifying crystallization step in which the bisphenol is precipitated out of the organic phase obtained in the water washing step, wherein the purifying crystallization step is performed after the water washing step is repeated until an electric conductivity of the water phase to be removed becomes 10 μS/cm or less.
<X3> The method for producing a bisphenol according to the above <12>, including: a water washing step in which the adsorption-purified liquid is mixed with water, the resultant is then separated into an organic phase containing the bisphenol and a water phase by phase separation, and the water phase is removed to obtain the organic phase; a concentration step in which an organic solvent is removed from the organic phase to obtain the bisphenol in a melt state; and a granulation step in which the bisphenol in a melt state is granulated to obtain a granulated product, wherein the concentration step is performed after the water washing step is repeated until an electric conductivity of the water phase to be removed becomes 10 μS/cm or less.
<X4> The method for producing a bisphenol according to the above <12>, further including a bisphenol synthesis step in which the bisphenol is obtained from a ketone or an aldehyde and an aromatic monoalcohol, the bisphenol synthesis step including the following step A to step D, wherein the adsorption-purified liquid is supplied to at least one of the following step A to the step D:

step A: a step in which the ketone or the aldehyde and the aromatic monoalcohol are subjected to dehydration

condensation in the presence of an acid catalyst to obtain a reaction liquid A containing the bisphenol;
step B: a step in which the unreacted ketone or aldehyde and water are distilled off from the reaction liquid A to obtain a concentrated liquid B;
step C: a step in which a slurry obtained by subjecting the concentrated liquid B to crystallization is separated into a mother liquor C and a cake c by solid-liquid separation; and
step D: a step in which the cake c is purified to obtain the bisphenol.

Advantageous Effect of Invention

The present invention makes it possible to provide a method for producing a bisphenol, by which a high-purity bisphenol can be obtained and a method for producing a recycled polycarbonate resin by using the bisphenol as a raw material.

Brief Description of Drawings

[Figure 1] Figure 1 is a flowchart of an example of a method for producing a bisphenol of the present invention.
[Figure 2] Figure 2 is a flowchart of another example of the method for producing a bisphenol of the present invention.
[Figure 3] Figure 3 is a flowchart of another example of the method for producing a bisphenol of the present invention.
[Figure 4] Figure 4 is a flowchart of another example of the method for producing a bisphenol of the present invention.
[Figure 5] Figure 5 is a more specific flowchart of the method for producing a bisphenol shown in Figure 4.

Description of Embodiments

Although embodiments of the present invention will be described in detail below, the following description about components is an example of the embodiments of the present invention. The present invention is not limited to the following description as long as the gist thereof is not exceeded. It should be noted that when a numerical range is herein expressed using "to", the numerical range includes numerical values or physical property values before and after "to".

<Method for Producing Bisphenol>

The present invention relates to a method for producing a bisphenol by removing, from a mixed solution containing a bisphenol and a resin other than a polycarbonate resin (hereinafter referred to as "another resin"), the another resin to obtain the bisphenol, the method including a step 1 in which the bisphenol is precipitated out of an aromatic monoalcohol-containing solution in which the bisphenol and the another resin are dissolved in a solvent containing an aromatic monoalcohol to obtain a slurry of the bisphenol, and the slurry of the bisphenol is then subjected to solid-liquid separation to obtain a crude cake of the bisphenol and a mother liquor (hereinafter sometimes referred to as "method for producing a bisphenol of the present invention").

When a bisphenol is obtained from a mixed solution containing the bisphenol and another resin, the obtained bisphenol is likely to be contaminated with the another resin, and therefore a high-purity bisphenol is difficult to obtain. However, the present inventors have found that even in the presence of another resin at the time of precipitation of the bisphenol, precipitation in the presence of an aromatic monoalcohol makes it possible to obtain a bisphenol while dissolved another resin is prevented from being attached to the bisphenol. Therefore, the method for producing a bisphenol of the present invention makes it possible to obtain a high-purity bisphenol. The obtained bisphenol has high polymerization activity, which makes it possible to obtain a polycarbonate resin being excellent in color hue and having a desired molecular weight.

The method for producing a bisphenol of the present invention includes the step of removing, from a mixed solution containing a bisphenol and another resin, the another resin to obtain the bisphenol. Further, the step of removing the another resin from the mixed solution to obtain a bisphenol includes a step 1. The step 1 is a crude crystallization step in which the bisphenol is precipitated out of an aromatic monoalcohol-containing solution in which the bisphenol and the another resin are dissolved in a solvent containing an aromatic monoalcohol to obtain a slurry of the bisphenol, and the slurry of the bisphenol is then subjected to solid-liquid separation to obtain a crude cake of the bisphenol and a mother liquor.

(Mixed Solution)

The mixed solution is a solution containing a bisphenol and another resin. For example, a decomposed liquid

obtained by depolymerizing a polycarbonate resin contained in a polycarbonate resin complex that will be described later can be used as a mixed solution.

In the present invention, the another resin is a resin other than a polycarbonate resin and refers to a polymer having a molecular weight of 10000 or more. Examples of the another resin contained in the mixed solution include polypropylene, polyethylene, polystyrene, an acrylic resin, polyethylene terephthalate, polybutylene terephthalate, an ABS resin, a polyamide, a phenol resin, a polyurethane, polylactic acid, and a silicone resin. In the present invention, the another resin includes at least another resin soluble in a solvent containing an aromatic monoalcohol. The another resin soluble in a solvent containing an aromatic monoalcohol is removed in the step 1. The another resin soluble in a solvent containing an aromatic monoalcohol is preferably at least one selected from the group consisting of an acrylic resin, polyethylene terephthalate, polybutylene terephthalate, an ABS resin, a polyamide, a phenol resin, a polyurethane, polylactic acid, and a silicone resin.

The mixed solution and the aromatic monoalcohol-containing solution may be the same or different depending on a solvent contained in the mixed solution. When the mixed solution contains an aromatic monoalcohol and the bisphenol and the another resin are dissolved, the mixed solution can be used as an aromatic monoalcohol-containing solution. In this case, the mixed solution may directly be used to precipitate the bisphenol or may be subjected to concentration, dilution, or removal of insoluble matter and then used to precipitate the bisphenol. When the mixed solution does not contain an aromatic monoalcohol, the aromatic monoalcohol-containing solution is prepared by replacing the solvent of the mixed solution with an aromatic monoalcohol.

The aromatic monoalcohol-containing solution out of which the bisphenol is to be precipitated is preferably a homogeneous solution. Therefore, when the mixed solution or the aromatic monoalcohol-containing solution contains insoluble matter, it is preferred that before the precipitation of the bisphenol, the mixed solution or the aromatic monoalcohol-containing solution is subjected to solid-liquid separation to obtain a homogeneous solution, and the bisphenol is precipitated out of the homogeneous solution. When the aromatic monoalcohol-containing solution is prepared by subjecting the mixed solution to solvent replacement, the solid-liquid separation may be performed before, after, or both before and after the solvent replacement.

It should be noted that the mixed solution may contain a resin insoluble in a solvent containing an aromatic monoalcohol in addition to the another resin soluble in a solvent containing an aromatic monoalcohol. The resin insoluble in a solvent containing an aromatic monoalcohol can be removed by, for example, solid-liquid separation such as filtration.

(Aromatic Monoalcohol-Containing Solution)

The aromatic monoalcohol-containing solution is a solution in which a bisphenol and another resin are dissolved in a solvent containing an aromatic monoalcohol.

(Bisphenol)

The bisphenol contained in the aromatic monoalcohol-containing solution is typically represented by the following formula (II).

[Chemical Formula 1]

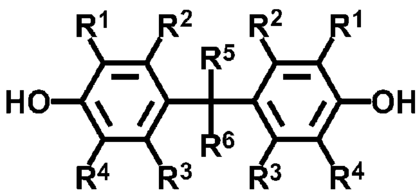

···(II)

In the formula (II), $R^1$ to $R^4$ are each independently a hydrogen atom, a halogen atom, an alkyl group, an alkoxy group, or an aryl group. Examples of these include a hydrogen atom, a fluoro group, a chloro group, a bromo group, a iodo group, a methyl group, an ethyl group, a n-propyl group, an i-propyl group, a n-butyl group, an i-butyl group, a t-butyl group, a n-pentyl group, an i-pentyl group, a n-hexyl group, a n-heptyl group, a n-octyl group, a n-nonyl group, a n-decyl group, a n-undecyl group, a n-dodecyl group, a methoxy group, an ethoxy group, a n-propoxy group, an i-propoxy group, a n-butoxy group, an i-butoxy group, a t-butoxy group, a n-pentyloxy group, an i-pentyloxy group, a n-hexyloxy group, a n-heptyloxy group, a n-octyloxy group, a n-nonyloxy group, a n-decyloxy group, a n-undecyloxy group, a n-dodecyloxy group, a cyclopropyl group, a cyclobutyl group, a cyclopentyl group, a cyclohexyl group, a cycloheptyl group, a cyclooctyl group, a cyclododecyl group, a benzyl group, a phenyl group, a tolyl group, and a 2,6-dimethylphenyl group.

In the formula (II), $R^5$ and $R^6$ are each independently a hydrogen atom, an alkyl group, an alkoxy group, or an aryl group. Examples of these include a hydrogen atom, a methyl group, an ethyl group, a n-propyl group, an i-propyl group, a n-butyl group, an i-butyl group, a t-butyl group, a n-pentyl group, an i-pentyl group, a n-hexyl group, a n-heptyl group, a n-octyl group, a 2-ethylhexyl group, a n-nonyl group, a n-decyl group, a n-undecyl group, a n-dodecyl group, a methoxy group, an ethoxy group, a n-propoxy group, an i-propoxy group, a n-butoxy group, an i-butoxy group, a t-butoxy group, a n-pentyloxy group, an i-pentyloxy group, a n-hexyloxy group, a n-heptyloxy group, a n-octyloxy group, a n-nonyloxy group, a n-decyloxy group, a n-undecyloxy group, a n-dodecyloxy group, a cyclopropyl group, a cyclobutyl group, a cyclopentyl group, a cyclohexyl group, a cycloheptyl group, a cyclooctyl group, a cyclododecyl group, a benzyl group, a phenyl group, a tolyl group, and a 2,6-dimethylphenyl group.

In the formula (II), $R^5$ and $R^6$ may be bonded or cross-linked to each other between the two groups to form a cycloalkylidene group, a fluorenylidene group (fluorene-9,9-diyl group), a xanthenylidene group (xanthene-9,9-diyl group), a thioxanthenylidene group (thioxanthene-9,9-diyl group), or the like. Examples of the cycloalkylidene group include cyclopropylidene, cyclobutylidene, cyclopentylidene, cyclohexylidene, 3,3,5-trimethylcyclohexylidene, cycloheptylidene, cyclooctylidene, cyclononylidene, cyclodecylidene, cycloundecylidene, and cyclododecylidene.

Specific examples of the bisphenol represented by the formula (II) include, but are not limited to, bisphenols such as 2,2-bis(4-hydroxyphenyl)propane, 2,2-bis(4-hydroxy-3-methylphenyl)propane, 2,2-bis(4-hydroxy-3,5-dimethylphenyl)propane, 1,1-bis(4-hydroxy-3-methylphenyl)cyclohexane, 9,9-bis(4-hydroxy-3-methylphenyl)fluorene, 3,3-bis(4-hydroxyphenyl)pentane, 3,3-bis(4-hydroxy-3-methylphenyl)pentane, 2,2-bis(4-hydroxyphenyl)pentane, 2,2-bis(4-hydroxy-3-methylphenyl) pentane, 3,3-bis(4-hydroxyphenyl)heptane, 3,3-bis(4-hydroxy-3-methylphenyl)heptane, 2,2-bis(4-hydroxyphenyl) heptane, 2,2-bis(4-hydroxy-3-methylphenyl) heptane, 4,4-bis(4-hydroxyphenyl)heptane, and 4,4-bis(4-hydroxy-3-methylphenyl)heptane.

Among these bisphenols, preferred is any one selected from the group consisting of 2,2-bis(4-hydroxyphenyl) propane, 2,2-bis(4-hydroxy-3-methylphenyl)propane, 2,2-bis(4-hydroxy-3,5-dimethylphenyl)propane, and 1,1-bis(4-hydroxy-3-methylphenyl)cyclohexane, and particularly preferred is 2,2-bis(4-hydroxyphenyl)propane (hereinafter referred to as "BPA" or "bisphenol A").

(Aromatic Monoalcohol)

Examples of the aromatic monoalcohol contained in the aromatic monoalcohol-containing solution include phenol, cresol, and xylenol. The aromatic monoalcohol preferably contains phenol and/or cresol and more preferably contains phenol.

The aromatic monoalcohol-containing solution may contain a solvent other than the aromatic monoalcohol as long as the bisphenol can be dissolved therein. The amount-of-substance ratio of the aromatic monoalcohol to the bisphenol (amount of substance (mol) of aromatic monoalcohol/amount of substance (mol) of bisphenol) may be set to, for example, 0.5 or more, 0.6 or more, or 0.7 or more and is preferably 1.0 or more. The upper limit of the amount of the aromatic monoalcohol contained in the aromatic monoalcohol-containing solution is not limited, but the amount-of-substance ratio is preferably 20 or less, more preferably 15 or less, even more preferably 10 or less because if the amount-of-substance ratio exceeds 20, the bisphenol is less likely to precipitate so that the yield of the bisphenol reduces. Therefore, the amount-of-substance ratio of the aromatic monoalcohol to the bisphenol is preferably 0.5 to 20, more preferably 1.0 to 20, even more preferably 1.0 to 15, particularly preferably 1.0 to 10.

(Precipitation of Bisphenol)

In the step 1, the bisphenol is first precipitated out of the aromatic monoalcohol-containing solution to obtain a slurry of the bisphenol. A method for precipitating the bisphenol is not limited and may be a general method for precipitating a crystal out of a solution. Preferred is a method for precipitating the bisphenol by cooling. Cooling conditions are not limited, but in order to efficiently precipitate the bisphenol, the cooling is preferably performed at a temperature lower by 20°C to 100°C than the temperature of the aromatic monoalcohol-containing solution to be subjected to the step 1. The aromatic monoalcohol-containing solution to be subjected to the step 1 is usually at a temperature of about 40°C to 120°C to prevent solidification etc. and is therefore preferably cooled to -20°C to 60°C, more preferably to 0°C to 50°C to precipitate the bisphenol. By cooling the aromatic monoalcohol-containing solution to such a temperature, a slurry having excellent fluidity can be obtained.

The bisphenol may be precipitated as a crystal of the bisphenol or a crystalline adduct (adduct crystal) of the bisphenol and a solvent. In the case of bisphenol A, it is preferred that phenol is used as an aromatic monoalcohol and a crystalline adduct of bisphenol A and phenol is precipitated to obtain a crude cake of the crystalline adduct. When bisphenol A is precipitated as a crystalline adduct, the another resin is less likely to attach to the precipitated crystal, thereby improving the efficiency of removing the another resin dissolved in a solvent containing phenol.

(Solid-Liquid Separation)

In the step 1, after the slurry of the bisphenol is obtained, the slurry of the bisphenol is subjected to solid-liquid separation to obtain a crude cake of the bisphenol and a mother liquor. The solid-liquid separation can be performed by a publicly-known means such as filtration or centrifugal separation. For example, the solid-liquid separation can be performed using a horizontal belt filter, a rotary vacuum filter, a rotary pressure filter, a centrifugal filtration separator, a centrifugal sedimentation separator, a centrifugal separator of hybrid type of these (screen bowl decanter), or the like.

(Step 2)

The method for producing a bisphenol of the present invention preferably includes, after the step 1, a step 2 in which a washing liquid is supplied to the crude cake of the bisphenol to wash the crude cake of the bisphenol, so that a purified cake of the bisphenol is obtained. By washing away the another resin etc. attached to the crude cake with a washing liquid, the purity of the bisphenol can further be improved.

(Washing Liquid)

The washing liquid may be at least one selected from the group consisting of an aromatic monoalcohol, an aliphatic monoalcohol, a ketone, an aromatic hydrocarbon, an aliphatic hydrocarbon, and water.
More specifically, the washing liquid is preferably at least one selected from the group consisting of phenol, cresol, methanol, ethanol, acetone, cyclohexanone, benzene, toluene, xylene, hexane, heptane, cyclohexane, and water.
The washing is performed at a temperature of about 10°C to 80°C, preferably 15°C or more, more preferably 20°C or more. The crude cake can be washed at a temperature within the above range by supplying a washing liquid heated to a desired temperature to the crude cake or by supplying a washing liquid to the crude cake heated to a desired temperature.
The amount of the washing liquid used per cycle of washing the crude cake of the bisphenol (amount of washing liquid used per cycle of washing (g)/mass of crude cake (g)) is preferably 0.5 or more, more preferably 1.0 or more.
A washing method may be, for example, a method in which the crude cake is subjected to filtration or centrifugal separation while a washing liquid is supplied thereto or a method in which the crude cake and a washing liquid are mixed and then the resultant is again subjected to solid-liquid separation. The washing may be performed twice or more, but is usually performed five times or less, preferably three times or less because if the number of times of washing is too large, the yield of the bisphenol reduces.
The method for producing a bisphenol of the present invention preferably includes a step in which the crude cake of the bisphenol or a purified cake obtained by washing the crude cake is dissolved in a solvent or melted to obtain a bisphenol solution, and the bisphenol solution is brought into contact with a solid adsorbent to obtain an adsorption-purified liquid. This makes it possible to obtain a higher-purity bisphenol.
It should be noted that a method for obtaining a purified cake by washing the crude cake is the same as that used in the step 2.
Hereinbelow, the method for producing a bisphenol of the present invention will be described in detail with reference to a case where a depolymerization step is performed before the step 1.

<Method for Producing Bisphenol (I)>

Figure 1 is a flowchart of an example of the method for producing a bisphenol of the present invention. A method for producing a bisphenol (I) shown in Figure 1 includes a depolymerization step, a step 1, and a step 2. By performing the step 1 and the step 2 after the depolymerization step as shown in the flowchart of Figure 1, a higher-purity bisphenol can be obtained. In the method for producing a bisphenol (I), the step 1 and the step 2 are performed as a post-process subsequent to the step of depolymerizing a polycarbonate resin contained in a polycarbonate resin complex containing the polycarbonate resin and another resin. Particularly, the step 1 and the step 2 are preferably performed after the depolymerization step using a waste polycarbonate resin complex.
When polycarbonate resin depolymerization is performed using, as a raw material, a polycarbonate resin complex containing a polycarbonate resin and another resin, a decomposed liquid after depolymerization contains not only a bisphenol but also the another resin so that the bisphenol to be obtained is likely to have low purity due to contamination with the another resin.
Conventionally, the decomposed liquid after depolymerization is generally purified by crystallization in the absence of an aromatic monoalcohol or after the content of an aromatic monoalcohol is reduced as much as possible by operation such as distillation and the aromatic monoalcohol is replaced with an organic solvent such as an aromatic hydrocarbon. It has been believed that obtaining a bisphenol cake by crystallization in the presence of an aromatic

monoalcohol after depolymerization is more trouble than it is worth because the bisphenol cake contains a large amount of the aromatic monoalcohol and therefore there is a fear that coloration occurs due to oxidation of the aromatic monoalcohol or because the aromatic monoalcohol is soluble in water and therefore the efficiency of water washing in an oil-water separation state is poor. However, the present inventors have found that when a bisphenol is crystallized in the presence of an aromatic monoalcohol after depolymerization of a polycarbonate resin contained in a polycarbonate resin complex containing the polycarbonate resin and another resin, the another resin can be prevented from being attached to a crystal of the bisphenol even in a case where the another resin is also present during crystallization, which makes it easy to separate the bisphenol and the another resin from each other.

The method for producing a bisphenol of the present invention makes it possible to obtain a high-purity bisphenol even when a polycarbonate resin complex containing a polycarbonate resin and another resin is used as a raw material, particularly even when a polycarbonate resin complex such as a coated polycarbonate resin molded body or a molded body of a polymer alloy of a polycarbonate resin and another resin is used as a raw material.

[Depolymerization Step]

The depolymerization step is a step performed before the step 1 and is specifically a step in which a polycarbonate resin (PC) contained in a polycarbonate resin complex (PC + P) containing the polycarbonate resin and another resin (P) is depolymerized to obtain a decomposed liquid (BP + P) containing a bisphenol and the another resin.

(Polycarbonate Resin (PC))

The polycarbonate resin contained in the polycarbonate resin complex used as a raw material for depolymerization contains a repeating structural unit including a bisphenol unit and a carbonate unit, and a typical example of such a polycarbonate resin is one having a structural unit represented by the following formula (I).

[Chemical Formula 2]

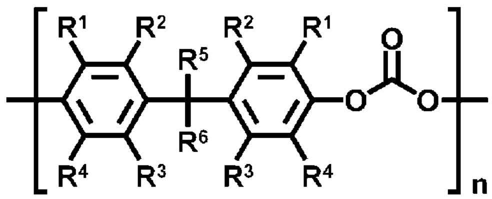

···(I)

$R^1$ to $R^6$ in the general formula (I) are the same as $R^1$ to $R^6$ in the above formula (II), respectively. In the general formula (I), n is the number of structural units. n is not limited but is, for example, 2 to 1000.

Specific examples of the structural unit represented by the above general formula (I) include, but are not limited to, structural units including a carbonate unit and a bisphenol unit derived from a bisphenol such as 2,2-bis(4-hydroxyphenyl)propane, 2,2-bis(4-hydroxy-3-methylphenyl)propane, 2,2-bis(4-hydroxy-3,5-dimethylphenyl)propane, 1,1-bis(4-hydroxy-3-methylphenyl)cyclohexane, 9,9-bis(4-hydroxy-3-methylphenyl)fluorene, 3,3-bis(4-hydroxyphenyl)pentane, 3,3-bis(4-hydroxy-3-methylphenyl)pentane, 2,2-bis(4-hydroxyphenyl)pentane, 2,2-bis(4-hydroxy-3-methylphenyl) pentane, 3,3-bis(4-hydroxyphenyl)heptane, 3,3-bis(4-hydroxy-3-methylphenyl)heptane, 2,2-bis(4-hydroxyphenyl) heptane, 2,2-bis(4-hydroxy-3-methylphenyl) heptane, 4,4-bis(4-hydroxyphenyl)heptane, or 4,4-bis(4-hydroxy-3-methylphenyl)heptane.

Among these, the polycarbonate resin preferably has a structural unit including a carbonate unit and a bisphenol unit derived from a bisphenol selected from the group consisting of 2,2-bis(4-hydroxyphenyl)propane, 2,2-bis(4-hydroxy-3-methylphenyl)propane, 2,2-bis(4-hydroxy-3,5-dimethylphenyl)propane, and 1,1-bis(4-hydroxy-3-methylphenyl)cyclohexane.

Particularly, a polycarbonate resin having a structural unit including a carbonate unit and a bisphenol unit derived from 2,2-bis(4-hydroxyphenyl)propane (hereinafter referred to as "BPA" or "bisphenol A") (a BPA-type polycarbonate resin having a structural unit represented by the above general formula (I) wherein $R^1$ to $R^4$ are each a hydrogen atom and $R^5$ and $R^6$ are each a methyl group) is easily depolymerized, and therefore in the method for producing a bisphenol of the present invention, BPA is preferably produced from a polycarbonate resin complex containing a BPA-type polycarbonate resin.

(Polycarbonate Resin Complex Containing Polycarbonate Resin and Another Resin)

The polycarbonate resin complex (hereinafter sometimes referred to as "PC complex") containing a polycarbonate resin and another resin may be a polycarbonate resin molded body containing another resin as an impurity, but is preferably a resin molded body containing a polycarbonate resin and another resin. Such a resin molded body cannot be separated into a polycarbonate resin and another resin by sorting and is therefore more suitable as a raw material used in the method for producing a bisphenol of the present invention which is capable of obtaining a bisphenol by removing another resin.
Examples of the resin molded body containing a polycarbonate resin and another resin include the following (c1) to (c5):

(c1) a molded body of a polymer alloy of a polycarbonate resin and another resin;
(c2) a polycarbonate resin molded body having a surface coated with another resin;
(c3) a polycarbonate resin molded body having a surface coated with a polymer alloy of a polycarbonate resin and another resin;
(c4) a resin molded body of a polymer alloy of a polycarbonate resin and another resin, the resin molded body having a surface coated with the another resin;
(c5) a resin molded body of a polymer alloy of a polycarbonate resin and another resin, the resin molded body having a surface coated with a polymer alloy of the polycarbonate resin and the another resin;
(c6) a resin molded body of a polymer alloy of a polycarbonate resin and another resin, the resin molded body having a surface coated with the polycarbonate resin;
(c7) a resin molded body of another resin, the resin molded body having a surface coated with a polycarbonate resin; and
(c8) a resin molded body of another resin, the resin molded body having a surface coated with a polymer alloy of a polycarbonate resin and the another resin.

It should be noted that the polymer alloy of a polycarbonate resin and another resin is a concept including both a mixture of a polycarbonate resin and another resin and a copolymer of a polycarbonate resin and another resin. The resin molded body containing a polycarbonate resin and another resin and the polycarbonate resin molded body may be used in combination.
Specific examples of these molded bodies include molded bodies used as housings of electronic devices and electronics, sundry goods, optical recording media, car head lump covers, illumination lamp covers, car interior materials, car exterior materials, transport containers, and building materials (e.g., carport roof materials and highway noise barriers).
Examples of the another resin contained in the PC complex include polypropylene, polyethylene, polystyrene, an acrylic resin, polyethylene terephthalate, polybutylene terephthalate, an ABS resin, a polyamide, a phenol resin, a polyurethane, polylactic acid, and a silicone resin. The PC complex contains at least another resin soluble in a solvent containing an aromatic monoalcohol, and such another resin is preferably at least one selected from the group consisting of an acrylic resin, polyethylene terephthalate, polybutylene terephthalate, an ABS resin, a polyamide, a phenol resin, a polyurethane, polylactic acid, and a silicone resin. These resins may be contained alone or in combination of two or more of them as another resin in the PC complex.
The polycarbonate resin complex is preferably waste plastic (waste material). The waste plastic containing a polycarbonate resin and another resin is a post-consumer material or a pre-consumer material. The post-consumer material is a material incorporated as molded bodies in various products and discarded after use by consumers. Examples of the pre-consumer material include molded bodies rejected based on the quality of ready-made products after coating or compounding. These post-consumer materials or pre-consumer materials can be used by appropriately crushing or pulverizing them.

(Depolymerization)

Examples of a depolymerization (decomposition) method include hydrolysis using water, phenolysis using phenol, alcoholysis using an alcohol, and aminolysis using an amine. In a general depolymerization method, a polycarbonate resin contained in a polycarbonate resin complex containing the polycarbonate resin and another resin is decomposed using a decomposition agent such as water, phenol, an alcohol, or an amine in the presence of a solvent and a catalyst. Therefore, any one of these depolymerization methods may be used.
It is preferred that after a reaction liquid is prepared as a slurry in which part of a polycarbonate resin is dissolved in a solvent, depolymerization is allowed to proceed while the polycarbonate resin is dissolved in the solvent. Even when the prepared reaction liquid is a slurry, the polycarbonate resin is dissolved as depolymerization proceeds. It should be noted

that depolymerization may proceed during reaction liquid preparation.

A temperature and a reaction temperature during depolymerization are not limited and may appropriately be set depending on the melting points, boiling points, etc. of a raw material and a decomposition agent to be used. For example, the temperature may be 10 to 200°C or 20 to 180°C, and the reaction time may be 0.1 to 30 hours, 0.5 to 25 hours, or 1 to 20 hours. The depolymerization may be performed under ordinary pressure or increased pressure.

(i) Method of depolymerization in presence of aromatic monoalcohol

From the viewpoint of ease of preparation of an aromatic monoalcohol-containing solution, depolymerization of a polycarbonate resin contained in a PC complex is preferably performed in the presence of an aromatic monoalcohol. Examples of a method for performing depolymerization in the presence of an aromatic monoalcohol include a method in which a polycarbonate resin is depolymerized in the presence of an aromatic monoalcohol and a catalyst and a method in which a polycarbonate resin is depolymerized in the presence of an aromatic monoalcohol, a catalyst, and a decomposition agent other than an aromatic monoalcohol.

The aromatic monoalcohol can function as a solvent or a decomposition agent. Examples of the aromatic monoalcohol include phenol, cresol, and xylenol. The aromatic monoalcohol preferably contains phenol or cresol. The aromatic monoalcohol is more preferably phenol because, in the step 1, a bisphenol such as bisphenol A can be precipitated as a crystalline adduct.

The catalyst for depolymerization is not limited as long as it can promote decomposition of a polycarbonate resin. Examples of such a catalyst for depolymerization include: alkali metal hydroxides such as sodium hydroxide and potassium hydroxide; alkali metal carbonates such as sodium carbonate, sodium hydrogen carbonate, potassium carbonate, and potassium hydrogen carbonate; alkylamines such as methylamine, ethylamine, propylamine, dimethylamine, diethylamine, and trimethylamine; inorganic acids such as hydrochloric acid, sulfuric acid, and phosphoric acid; and organic acids such as carboxylic acid and sulfonic acid.

When the mass ratio of the catalyst to the PC complex (mass of catalyst/mass of PC complex) is low, a decomposition time increases due to a reduction in decomposition speed so that efficiency tends to deteriorate. For this reason, the mass ratio is preferably 0.001 or more, more preferably 0.005 or more, even more preferably 0.01 or more. On the other hand, when the mass ratio is high, the amount of an acid or a base required for neutralization tends to increase. For this reason, the mass ratio is preferably 50 or less, more preferably 20 or less, even more preferably 10 or less.

Examples of the decomposition agent other than an aromatic monoalcohol include water, an aliphatic monoalcohol, and an amine. Examples of the aliphatic monoalcohol include methanol, ethanol, n-propanol, i-propanol, n-butanol, i-butanol, t-butanol, n-pentanol, i-pentanol, n-hexanol, n-heptanol, n-octanol, n-nonanol, n-decanol, n-undecanol, and n-dodecanol. The aliphatic monoalcohol is preferably an alcohol having 1 to 5 carbon atoms and is more preferably any one selected from the group consisting of methanol, ethanol, and butanol.

(ii) Method of depolymerization in absence of aromatic monoalcohol

The depolymerization may be performed in the absence of an aromatic monoalcohol. An example of a method for performing depolymerization in the absence of an aromatic monoalcohol is a method in which a polycarbonate resin contained in a PC complex is depolymerized in the presence of a solvent other than an aromatic monoalcohol, a catalyst, and a decomposition agent other than an aromatic monoalcohol.

The solvent other than an aromatic monoalcohol is not limited as long as it can dissolve a polycarbonate resin, and examples thereof include: aromatic hydrocarbons such as benzene, toluene, and xylene; halogen-based solvents such as methylene chloride and the like; and dialkyl carbonates such as dimethyl carbonate, diethyl carbonate, dipropyl carbonate, and dibutyl carbonate. An aliphatic monoalcohol functions as a decomposition agent and a solvent and therefore may be used as a decomposition agent and a solvent by excessively supplying it as compared to when used only as a decomposition agent. Considering recycling, a solvent having a boiling point of 200°C or less is preferably used.

When the mass ratio of the solvent to the PC complex (mass of solvent/mass of PC complex) is low, the time require to produce a bisphenol tends to increase due to a reduction in the dissolution speed of the polycarbonate resin. For this reason, the mass ratio is preferably 0.01 or more, more preferably 0.03 or more, even more preferably 0.05 or more. On the other hand, when the mass ratio is high, the amount of the polycarbonate resin that can be decomposed at a time reduces due to an increase in the volume of the solvent relative to a decomposition tank so that pot efficiency tends to deteriorate. For this reason, the mass ratio is preferably 100 or less, more preferably 70 or less, even more preferably 50 or less.

The catalyst and the decomposition agent other than an aromatic monoalcohol may be the same as those used in the method of depolymerization in the presence of an aromatic monoalcohol (i).

A preferred example of the method of depolymerization in the absence of an aromatic monoalcohol is a method in which depolymerization is performed using a dialkyl carbonate as a solvent and an aliphatic monoalcohol as a

decomposition agent. The dialkyl carbonate and the aliphatic monoalcohol are preferably the same in the number of carbon atoms of their alkyl group. More preferably, the dialkyl carbonate and the aliphatic monoalcohol have the same alkyl group having 1 to 5 carbon atoms. For example, a combination of dimethyl carbonate and methanol is preferred.

(Mixed Solution)

In the method for producing a bisphenol (I), the mixed solution is a decomposed liquid obtained by depolymerizing a polycarbonate resin contained in a polycarbonate resin complex containing the polycarbonate resin and another resin.

(Bisphenol)

The bisphenol contained in the decomposed liquid is one obtained by depolymerizing a polycarbonate resin. The bisphenol has a structure corresponding to the bisphenol unit of the polycarbonate resin. When a resin molded body containing a polycarbonate resin having a structural unit represented by the above formula (I) is subjected to depolymerization, a bisphenol represented by the following formula (II) is obtained.

[Chemical Formula 3]

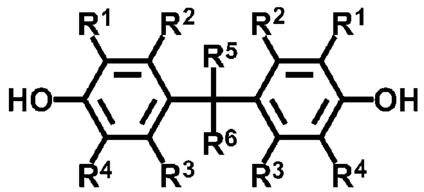

···(II)

$R^1$ to $R^6$ in the general formula (II) are the same as $R^1$ to $R^6$ in the above formula (I), respectively.

(Preparation of Aromatic Monoalcohol-Containing Solution)

In the decomposed liquid, a bisphenol generated by depolymerization of a polycarbonate resin is dissolved. Further, the decomposed liquid contains another resin contained in the PC complex, and at least part of the another resin is dissolved in the decomposed liquid. Further, the decomposed liquid contains a solvent, a catalyst, etc. used for the depolymerization. Depending on the composition of the decomposed liquid, the decomposed liquid may directly be used in the step 1 or an aromatic monoalcohol-containing solution prepared from the decomposed liquid may be used in the step 1.

When the depolymerization is performed in the presence of an aromatic monoalcohol, the decomposed liquid contains a bisphenol, another resin contained in the PC complex, and the aromatic monoalcohol. In such a case where the decomposed liquid contains an aromatic monoalcohol, the decomposed liquid may directly be used as an aromatic monoalcohol-containing solution in the step 1. Further, the aromatic monoalcohol-containing solution may be subjected to dilution or concentration before use in order to adjust the amount-of-substance ratio between the bisphenol and the aromatic monoalcohol or the degree of solubility of the bisphenol. The dilution may be performed using an aromatic monoalcohol or a solvent other than an aromatic monoalcohol. That is, the mixed solution is one obtained by performing depolymerization in the presence of an aromatic monoalcohol in the depolymerization step, and the mixed solution is an aromatic monoalcohol-containing solution. In this case, the aromatic monoalcohol used in the depolymerization step corresponds to the aromatic monoalcohol contained in the aromatic monoalcohol-containing solution.

When the depolymerization is performed in the absence of an aromatic monoalcohol, the decomposed liquid contains a bisphenol, another resin contained in the PC complex, and a solvent other than an aromatic monoalcohol and therefore does not contain an aromatic monoalcohol. In such a case where the decomposed liquid does not contain an aromatic monoalcohol, an aromatic monoalcohol-containing solution is prepared by adding an aromatic monoalcohol. For example, when the decomposed liquid does not contain an aromatic monoalcohol, an aromatic monoalcohol-containing solution can be prepared by solvent replacement. That is, the mixed solution is one obtained by performing depolymerization in the presence of a solvent other than an aromatic monoalcohol in the depolymerization step, and the aromatic monoalcohol-containing solution is one obtained by replacing the solvent of the obtained mixed solution with an aromatic monoalcohol. The solvent replacement may be performed by replacing part or all of the solvent of the mixed solution. After the solvent replacement, dilution or concentration may be performed to adjust the amount-of-substance ratio between the bisphenol and the aromatic monoalcohol or the degree of solubility of the bisphenol. The dilution may be performed using an aromatic monoalcohol or a solvent other than an aromatic monoalcohol.

From the viewpoint of sufficiently obtaining the effect of preventing attachment of the another resin, the amount of substance of the aromatic monoalcohol relative to the amount of substance of the bisphenol in the aromatic monoalcohol-containing solution is preferably 0.5 or more, more preferably 0.6 or more, even more preferably 0.7 or more, particularly preferably 1.0 or more. If the amount of substance of the aromatic monoalcohol is too large, the bisphenol is less likely to precipitate so that the yield of the bisphenol reduces. Therefore, the amount-of-substance ratio of the aromatic monoalcohol to the bisphenol is preferably 20 or less, more preferably 15 or less, even more preferably 10 or less. That is, the amount of substance of the aromatic monoalcohol relative to the amount of substance of the bisphenol in the aromatic monoalcohol-containing solution is preferably 0.5 to 20, more preferably 0.6 to 20, even more preferably 0.7 to 15, particularly preferably 1.0 to 10.

A method for precipitating a bisphenol and a solid-liquid separation method used in the step 1 and the type of washing liquid to be used and washing conditions in the step 2 are as described above.

The decomposed liquid often contains insoluble matter. Therefore, before precipitation of the bisphenol, the mixed solution (decomposed liquid) or the aromatic monoalcohol-containing solution is preferably subjected to solid-liquid separation to obtain a homogeneous solution. That is, a visually-transparent aromatic monoalcohol-containing solution is preferably used in the step 1. The solid-liquid separation can be performed by a publicly-known means such as filtration or centrifugal separation. For example, the solid-liquid separation can be performed using a horizontal belt filter, a rotary vacuum filter, a rotary pressure filter, a centrifugal filtration separator, a centrifugal sedimentation separator, a centrifugal separator of hybrid type of these (screen bowl decanter), or the like. When part of the another resin is insoluble matter, such another resin is removed by the solid-liquid separation.

A specific example of the method for producing a bisphenol (I) in which an aromatic monoalcohol-containing solution being a homogeneous solution is used is a method including a depolymerization step, a solution preparation step, a step 1A, and a step 2 described below:

depolymerization step: a step in which a polycarbonate resin contained in a polycarbonate complex containing the polycarbonate resin and another resin is depolymerized to obtain a decomposed liquid containing a bisphenol and the another resin;

solution preparation step: a step in which a homogeneous solution containing the bisphenol, an aromatic monoalcohol, and the another resin is prepared using the decomposed liquid obtained in the depolymerization step;

step 1A: a step in which the bisphenol is precipitated out of the homogeneous solution obtained in the solution preparation step to obtain a slurry of the bisphenol, and the slurry of the bisphenol is then subjected to solid-liquid separation to obtain a crude cake of the bisphenol and a mother liquor; and

step 2: a step in which a washing liquid is supplied to the crude cake of the bisphenol, and the crude cake of the bisphenol is washed to obtain a purified cake of the bisphenol.

In this method, a homogeneous solution containing a bisphenol, an aromatic monoalcohol, and another resin other than a polycarbonate resin is prepared from a decomposed liquid obtained in the depolymerization step and is used as an aromatic monoalcohol-containing solution in the step 1. The depolymerization step and the step 2 in this method are the same as those described above. The step 1A is also the same as the step 1 described above except that the homogeneous solution obtained in the solution preparation step is used as an aromatic monoalcohol-containing solution.

(Solution Preparation Step)

The homogeneous solution used in the step 1A is prepared from the decomposed liquid obtained in the depolymerization step. When the decomposed liquid contains an aromatic monoalcohol, the prepared homogeneous solution may directly be used in the step 1A without being subjected to concentration, dilution, or the like or may appropriately be concentrated or diluted before use in the step 1A. When the decomposed liquid does not contain an aromatic monoalcohol, a homogeneous solution may be prepared by adding an aromatic monoalcohol and, if necessary, concentrating the resultant. When the homogeneous solution is prepared, a solvent other than an aromatic monoalcohol may be added to adjust the degree of solubility of the bisphenol.

As described above, the amount of substance of the aromatic monoalcohol relative to the amount of substance of the bisphenol in the homogeneous solution is preferably 0.5 to 20, more preferably 0.6 to 20, even more preferably 0.7 to 15, particularly preferably 1.0 to 10. The homogeneous solution is preferably prepared by concentrating the bisphenol-containing decomposed liquid obtained in the depolymerization step or adding an aromatic monoalcohol to the bisphenol-containing decomposed liquid in such a manner that the amount-of-substance ratio of the aromatic monoalcohol to the bisphenol falls within the above range.

The decomposed liquid is subjected to solid-liquid separation to remove impurities so that a homogeneous solution is obtained. In the solution preparation step, the decomposed liquid is preferably filtered before use in the step 1A. A filtrate obtained by filtering the decomposed liquid may be concentrated before use, if necessary. When containing an

aromatic monoalcohol, the filtrate can be used in the step 1A. When the filtrate does not contain an aromatic monoalcohol, a homogeneous solution prepared by adding an organic solvent containing an aromatic monoalcohol to the filtrate can be used in the step 1A. When the decomposed liquid does not contain an aromatic monoalcohol, a homogeneous solution may be prepared by adding an organic solvent containing an aromatic monoalcohol to the decomposed liquid and then filtering the resultant. When insoluble matter remains in the decomposed liquid, the insoluble matter can be removed by filtration. When the PC complex contains another resin insoluble in an aromatic monoalcohol, such another resin can be removed by filtration. For example, polypropylene, polyethylene, polystyrene, and the like are insoluble in an aromatic monoalcohol and are therefore removed by filtration.

(Bisphenol)

The bisphenol contained in the homogeneous solution is one obtained by subjecting a PC complex containing a polycarbonate resin and another resin to depolymerization. The bisphenol has a structure corresponding to the bisphenol unit of the polycarbonate resin and is the same as that described above.

(Aromatic Monoalcohol)

Examples of the aromatic monoalcohol contained in the homogeneous solution include phenol, cresol, and xylenol, and phenol is preferred. When the depolymerization step is performed in the presence of an aromatic monoalcohol, the aromatic monoalcohol used in the depolymerization step usually corresponds to the aromatic monoalcohol contained in the homogeneous solution.

(Another Resin)

The another resin contained in the homogeneous solution is a polymer having a molecular weight of 10000 or more and refers to a polymer compound dissolved in the homogeneous solution. The another resin contained in the homogeneous solution is usually derived from another resin contained in the PC complex used as a raw material for depolymerization. For example, when a resin molded body containing a polycarbonate resin and another resin is subjected to depolymerization, another resin constituting the resin molded body and soluble in a solvent containing an aromatic monoalcohol corresponds to the another resin contained in the homogeneous solution. Specifically, the another resin contained in the homogeneous solution may be at least one selected from the group consisting of an acrylic resin, polyethylene terephthalate, polybutylene terephthalate, an ABS resin, a polyamide, a phenol resin, a polyurethane, polylactic acid, and a silicone resin.

(Homogeneous Solution)

The homogeneous solution to be subjected to the step 1 is a visually-transparent solution containing a bisphenol obtained by depolymerization and another resin derived from the PC complex. The homogeneous solution may contain a component other than a bisphenol, an aromatic monoalcohol, and another resin, such as a catalyst for depolymerization, a salt, or a decomposition agent.

It should be noted that insoluble matter contained in the mixed solution (decomposed liquid) or the aromatic monoalcohol-containing solution is difficult to visually confirm. Therefore, the mixed solution (decomposed liquid) or the aromatic monoalcohol-containing solution (especially, the aromatic monoalcohol-containing solution) is preferably filtered (passed through a filter) or centrifuged before use in the step 1 or the step 1A regardless of whether or not the mixed solution (decomposed liquid) or the aromatic monoalcohol-containing solution is a homogeneous solution (visually transparent).

When the bisphenol is precipitated as a crystalline adduct (adduct crystal), the another resin is less likely to attach to the precipitated crystal, thereby improving the efficiency of removing the another resin. Therefore, it is preferred that phenol is used as an aromatic monoalcohol, a crystalline adduct of the bisphenol and phenol is precipitated to obtain a crude cake of the crystalline adduct of the bisphenol and phenol in the step 1 or the step 1A, and a purified cake of the crystalline adduct of the bisphenol and phenol is obtained in the step 2. It is more preferred that phenol is used as an aromatic monoalcohol, a crystalline adduct of bisphenol A and phenol is precipitated to obtain a crude cake of the crystalline adduct of bisphenol and phenol in the step 1 or the step 1A, and a purified cake of the crystalline adduct of bisphenol A and phenol is obtained in the step 2.

<Method for Producing bisphenol (II)>

Figure 2 is a flowchart of another example of the method for producing a bisphenol of the present invention. A

method for producing a bisphenol (II) shown in Figure 2 includes a depolymerization step, a step 1, a step 2, an adsorption purification step, a water washing step, and a purifying crystallization step.

The depolymerization step, the step 1, and the step 2 are the same as those in the method for producing a bisphenol (I).

[Adsorption Purification Step]

In the method for producing a bisphenol (II), the adsorption purification step is a step performed after the step 2. Specifically, the adsorption purification step is a step in which the purified cake is dissolved in a solvent or melted to obtain a bisphenol solution, and then the bisphenol solution is brought into contact with a solid adsorbent to obtain an adsorption-purified liquid. By purifying the bisphenol solution with a solid adsorbent in such a manner as described above, impurities such as a colored component etc. can efficiently be removed. Since the another resin has been removed in the step 1 and the step 2, filterability or liquid permeability during contact with a solid adsorbent is excellent.

(Bisphenol Solution)

In the method for producing a bisphenol (II), the bisphenol solution is a liquid obtained by dissolving the purified cake in a solvent or melting the purified cake. The solvent of the bisphenol solution mainly contains an organic solvent but may contain water to the extent that it can dissolve the bisphenol. The organic solvent used to prepare the bisphenol solution is not limited as long as it can dissolve the bisphenol, and may be an aromatic hydrocarbon, an aromatic monoalcohol, a halogen-based solvent, a dialkyl carbonate, or the like. Among these, the organic solvent preferably contains at least one selected from the group consisting of benzene, toluene, xylene, phenol, cresol, methylene chloride, dimethyl carbonate, and diethyl carbonate.

When the amount of the organic solvent contained in the bisphenol solution is small, the liquid viscosity of the bisphenol solution increases in proportion to the concentration of the bisphenol. When the liquid viscosity is high, there is a possibility that during contact with a solid adsorbent, the bisphenol is precipitated and stuck to the solid adsorbent. Further, there is a fear that when the bisphenol solution is brought into contact with a solid adsorbent by a percolation method that will be described later, a device is broken due to a high differential pressure.
Therefore, the amount of the organic solvent contained in the bisphenol solution is preferably 20% by mass or more. The amount of the organic solvent contained in the bisphenol solution can freely be adjusted to, for example, 30% by mass or more, 40% by mass or more, or 50% by mass or more depending on the type of organic solvent to be used etc.

When the purified cake is composed of a crystalline adduct of the bisphenol and phenol, a bisphenol solution in which the bisphenol is dissolved in phenol can be obtained by thermally melting the crystal.

(Solid Adsorbent)

The solid adsorbent is preferably one that has a large specific surface area and efficiently adsorbs a colored component or one that has pores and adsorbs metal atoms such as sodium and potassium. Examples of such a solid adsorbent include activated clay, acid clay, activated carbon, and a cation exchange resin. These solid adsorbents may be used alone or in combination of two or more of them. The solid adsorbent preferably contains any one selected from the group consisting of activated clay, acid clay, activated carbon, and a cation exchange resin and more preferably contains any one of these as a main component (50% by mass or more of the solid adsorbent). Further, for the purpose of improving filterability or assisting dehydration etc., any of adsorbent selected from the group consisting of activated clay, acid clay, activated carbon, and a cation exchange resin may be used in combination with a general adsorbent such as molecular sieve, silica gel, alumina, cerite, or zeolite.

(Method for Bringing Bisphenol Solution into Contact with Solid Adsorbent)

A method for bringing the bisphenol solution into contact with a solid adsorbent is not limited, but as general methods, there are a contact method in which a solid adsorbent is charged into the bisphenol solution and sufficiently brought into contact with the bisphenol solution by stirring and is then removed by filtration and a percolation method in which the bisphenol solution is passed through a column filled with a solid adsorbent.

If the amount of the solid adsorbent supplied in the contact method is too small, there is a fear that a colored component cannot adequately be adsorbed. Therefore, the amount of the solid adsorbent to be supplied is preferably 0.01% by mass or more, more preferably 0.05% by mass or more, even more preferably 0.1% by mass or more relative to the amount of the bisphenol to be treated. On the other hand, if the amount of the solid adsorbent supplied is too large, a colored component can adequately be adsorbed, but load on the filtration process may increase or economic efficiency may deteriorate. Therefore, the amount of the solid adsorbent to be supplied is preferably 20% by mass or less, more

preferably 10% by mass or less, even more preferably 5% by mass or less relative to the amount of the bisphenol dissolved in the bisphenol solution. That is, the amount of the solid adsorbent to be supplied is preferably 0.01 to 20% by mass, more preferably 0.05 to 10% by mass, even more preferably 0.1 to 5% by mass relative to the amount of the bisphenol.

The amount of the solid adsorbent filled in the column in the percolation method is not limited as long as it is excessive relative to the amount of the bisphenol to be treated. Under the assumption that the solid adsorbent is repeatedly used, the amount of the solid adsorbent may be, for example, 10 times or more, 100 times or more, 1000 times or more relative to the amount of the bisphenol to be treated.

The adsorption purification step in which the bisphenol solution is brought into contact with a solid adsorbent is preferably performed in an inert gas atmosphere. By bringing the bisphenol solution into contact with a solid adsorbent in an inert gas atmosphere typified by nitrogen or argon, the color hue of the bisphenol solution can be maintained.

The temperature at the time when the bisphenol solution is brought into contact with a solid adsorbent is appropriately set depending on the type of solid adsorbent to be used. In order to more efficiently remove a colored component by adsorption, it is preferred that the bisphenol is completely dissolved in the bisphenol solution. Thus, the temperature should be set in consideration of the degree of solubility of the bisphenol in the bisphenol solution. Therefore, the temperature at the time when the bisphenol solution is brought into contact with a solid adsorbent is preferably 0°C to 200°C, more preferably 10°C to 190°C, even more preferably 20°C to 180°C.

The time during which the bisphenol solution is brought into contact with a solid adsorbent depends on the type and amount of solid adsorbent to be used and temperature, but may usually be 0.1 hours or more because if the time is short, adsorption of a colored component is inadequate. If the time is too long, the duration of occupancy of a device increases so that production efficiency deteriorates, and therefore the time is usually 100 hours or less. When the percolation method is used, a treatment time is controlled by the inner diameter and length of the column and the flow rate of a liquid passing through the column. The inner diameter and length of the column preferably have a margin relative to the amount of the bisphenol to be treated. Specifically, the inner diameter is preferably about 10 mm to 1000 mm, the length is preferably 20 mm to 3000 mm, and the flow rate is preferably 1 to 1000 mL/min.

(Adsorption-Purified Liquid)

The adsorption-purified liquid is a bisphenol solution after contact with a solid adsorbent. The adsorption-purified liquid obtained through treatment with a solid adsorbent has a reduced amount of impurities as comparted to the bisphenol solution before the treatment.

[Water Washing Step]

The water washing step is a step in which the adsorption-purified liquid obtained in the adsorption purification step is mixed with water, the resultant is then separated into an organic phase including the bisphenol and a water phase by phase separation, and the water phase is removed to obtain the organic phase. If the electric conductivity of the water phase is not 10 μS/cm or less, there is a possibility that the thermal stability of the bisphenol is impaired, and therefore the water washing step is repeatedly performed until the electric conductivity of the water phase to be removed becomes 10 μS/cm or less. Specifically, when the electric conductivity of the water phase removed exceeds 10 μS/cm, water is again supplied to the organic phase after removal of the water phase, the resultant is separated into an organic phase and a water phase by phase separation, and the water phase is removed. By repeating such operation, the electric conductivity of the water phase can be reduced.

The water to be supplied may be, for example, distilled water, demineralized water, ion-exchanged water, or pure water. In order to efficiently wash the adsorption-purified liquid, the electric conductivity of water to be supplied is preferably low as much as possible. For example, water having an electric conductivity of 5 μS/cm or less or 2 μS/cm or less is preferably used.

[Purifying Crystallization Step]

The purifying crystallization step is a step in which the bisphenol is precipitated out of the organic phase obtained in the water washing step. The purifying crystallization step can be performed in the same manner as in the step 1. Specifically, the bisphenol is precipitated by cooling the organic phase, and a slurry containing the precipitated bisphenol is separated into the bisphenol and a purifying crystallization mother liquor by solid-liquid separation to obtain the bisphenol. This makes it possible to obtain a solid bisphenol.

The obtained bisphenol may be dried, if necessary. A drying method is not limited but may be, for example, a method in which the bisphenol is heated under reduced pressure to distill off the organic solvent. In the method for producing a bisphenol (II), since the another resin has efficiently been removed in the step 1 and the step 2, the bisphenol can be dried by sufficiently vaporizing the solvent.

It should be noted that when the organic phase obtained in the water washing step contains phenol, whether the bisphenol such as bisphenol A is precipitated as a single crystal of the bisphenol or as a crystalline adduct of the bisphenol and phenol depends on the composition of the organic phase. Therefore, when a bisphenol that forms a crystalline adduct with phenol is produced, the composition of the bisphenol solution used in the adsorption purification step or the composition of the organic phase after the water washing step needs to be adjusted depending on the structure of a crystal to be obtained in the purifying crystallization step.

When the bisphenol that forms a crystalline adduct with phenol needs to be obtained as a crystalline adduct, the bisphenol should be precipitated in a state where phenol is present in the organic phase in an amount of 1.0 mol or more relative to the amount of the bisphenol. When the amount of phenol is small, the concentration of the slurry increases so that trouble such as scaling in a crystallizer may be caused. Therefore, the amount of phenol in the organic phase is preferably 2.0 mol or more, more preferably 3.0 mol or more relative to the amount of the bisphenol.

For example, when the purified cake obtained in the step 2 is a cake of a crystalline adduct, the cake is melted or phenol is added to the cake to obtain a bisphenol solution. At this time, the amount-of-substance ratio of phenol to the bisphenol is adjusted to 1.0 mol or more. This bisphenol solution is subjected to the adsorption purification step, and then the water washing step is performed. The thus obtained organic phase is cooled to precipitate a crystal so that a crystalline adduct can be obtained.

When the bisphenol that forms a crystalline adduct with phenol needs to be obtained as a single crystal, the composition of the bisphenol solution obtained in the adsorption purification step or the composition of the organic phase after the water washing step should be adjusted using an organic solvent other than phenol in such a manner that the organic solvent other than phenol is excessively present relative to phenol when the bisphenol is precipitated. The amount-of-substance ratio of the organic solvent other than phenol to phenol may be, for example, 5.0 mol or more or 6.0 mol or more.

When the purified cake obtained in the step 2 is a cake of a crystalline adduct, a single crystal of the bisphenol can be obtained by, for example, dissolving the purified cake in an aromatic hydrocarbon to prepare a bisphenol solution and performing the adsorption purification step, the water washing step, and the purifying crystallization step using this bisphenol solution. Before the purified cake is dissolved in an aromatic hydrocarbon, phenol may be distilled off. In this case, it is preferred that phenol is distilled off until the amount of phenol becomes less than 1.0 mol relative to the amount of the bisphenol.

When the adsorption purification step is performed in a state where a large amount of phenol is present by using, for example, a liquid obtained by melting the purified cake of a crystalline adduct as a bisphenol solution, a single crystal of the bisphenol can be obtained by, for example, adding an aromatic hydrocarbon to the organic phase after the water washing step to adjust the composition of the organic phase and then precipitating the bisphenol. Examples of a method for adjusting the composition of the organic phase include a method in which an aromatic hydrocarbon is supplied after phenol is distilled off until the amount of phenol becomes less than 1.0 mol relative to the amount of the bisphenol in the organic phase and a method in which the amounts of phenol, water, and an aromatic hydrocarbon are adjusted to achieve a certain composition. The amount of water is preferably 1.0 mol or more, more preferably 1.2 mol or more relative to the amount of phenol. The amount of an aromatic hydrocarbon is preferably 5.0 mol or more, more preferably 6.0 mol or more relative to the amount of phenol. It is estimated that when water is present in an equimolar amount or more relative to the amount of phenol, formation of a crystalline adduct of the bisphenol and phenol is prevented due to hydration of phenol and that when a large amount of an aromatic hydrocarbon having a low solubility of the bisphenol is present, precipitation of a bisphenol crystal can be promoted.

<Method for Producing Bisphenol (III)>

Figure 3 is a flowchart of another example of the method for producing a bisphenol of the present invention. A method for producing a bisphenol (III) shown in Figure 3 includes a depolymerization step, a step 1, a step 2, an adsorption purification step, a water washing step, a concentration step, and a granulation step.

The depolymerization step, the step 1, the step 2, the adsorption purification step, and the water washing step are the same as those in the method for producing a bisphenol (II).

[Concentration Step]

The concentration step is a step in which the organic solvent is removed from the organic phase obtained in the water washing step to obtain the bisphenol in a melt state. The concentration step is performed after performing the water washing step repeatedly until the electric conductivity of the water phase to be removed becomes 10 μS/cm or less.

Specifically, in the concentration step, the organic solvent is removed by subjecting the organic phase to distillation to obtain the bisphenol. The distillation may be performed at ordinary pressure, but reduced-pressure distillation is preferred. For example, the reduced-pressure distillation is performed at a temperature of 150°C to 250°C and a

pressure of 5 kPa to 80 kPa. The bisphenol taken out of a distillator is in a melt state and therefore can directly be used in the next step.

[Granulation Step]

The granulation step is a step in which the bisphenol in a melt state is granulated to obtain a granulated product. By feeding the bisphenol in a melt state (molten bisphenol) to a granulation tower or a flaker and granulating it, a solid bisphenol is obtained as prills or flakes. For example, when the molten bisphenol is supplied to a granulation tower so as to be granulated, the molten bisphenol is fed to the top of the granulation tower and sprayed through a plurality of holes provided in a nozzle plate disposed at the top of the tower. The sprayed molten bisphenol is cooled by a circulating gas moving upward from the bottom of the granulation tower and is taken out from the bottom of the tower as solid particles called prills.

<Method for Producing Bisphenol (IV)>

In the method for producing a bisphenol of the present invention, the bisphenol obtained by depolymerization may be purified and recovered using an existing or new production plant for continuously synthesizing a bisphenol from a ketone or an aldehyde and an aromatic monoalcohol. An example of such a production method is shown in Figure 4.

A method for producing a bisphenol (IV) shown in Figure 4 includes, in addition to a depolymerization step, a step 1, a step 2, and an adsorption purification step, a bisphenol synthesis step in which a bisphenol is obtained from a ketone or an aldehyde and an aromatic monoalcohol and a step X in which an adsorption-purified liquid obtained in the adsorption purification step is supplied to the bisphenol synthesis step.

The depolymerization step, the step 1, the step 2, and the adsorption purification step are the same as those in the method for producing a bisphenol (II).

[Bisphenol Synthesis Step]

The bisphenol synthesis step is a step of obtaining a bisphenol from a ketone or an aldehyde and an aromatic monoalcohol, and includes the following step A to step D:

step A: a step in which a ketone or an aldehyde and an aromatic monoalcohol are subjected to dehydration condensation in the presence of an acid catalyst to obtain a reaction liquid A containing a bisphenol;
step B: a step in which the unreacted ketone or aldehyde and water are distilled off from the reaction liquid A to obtain a concentrated liquid B;
step C: a step in which a slurry obtained by subjecting the concentrated liquid B to crystallization is separated into a mother liquor C and a cake c by solid-liquid separation; and
step D: a step in which the cake c is purified to obtain the bisphenol.

In some production plants for continuously synthesizing a bisphenol from a ketone or an aldehyde and an aromatic monoalcohol, the above step A to step D are performed. The method for producing a bisphenol can be performed using such a production plant in which the above step A to step D are performed. It should be noted that the bisphenol produced in the bisphenol synthesis step is the same as the bisphenol contained in the adsorption-purified liquid.

[Step X]

The step X is a step of supplying the adsorption-purified liquid to the bisphenol synthesis step. The adsorption-purified liquid is supplied to at least one of the step A to the step D in the bisphenol synthesis step. By doing so, it is possible to purify and recover the bisphenol contained in the adsorption-purified liquid together with a bisphenol produced from a ketone or an aldehyde and an aromatic monoalcohol. The adsorption-purified liquid may be supplied to one of the step A to the step D in the bisphenol synthesis step or may be supplied to two or more of these steps. The adsorption-purified liquid is preferably supplied to one or more of the steps A, B, and C.

Alternatively, a concentrated product obtained by subjecting the adsorption-purified liquid to distillation or crystallization to concentrate the bisphenol may be supplied to one or more of the steps included in the bisphenol synthesis step, or a product obtained by replacing the solvent of the concentrated product with a different solvent may be supplied to one or more of the steps included in the bisphenol synthesis step.

<Method for Producing Bisphenol (V)>

Figure 5 is a more specific flowchart of the method for producing a bisphenol shown in Figure 4. Figure 5 shows a method in which bisphenol A (BPA) is produced by the method for producing a bisphenol (V) shown in Figure 4 using, as a raw material for depolymerization, a waste resin molded body (BPA-type PC + p) containing a polycarbonate resin (BPA-type PC) having a bisphenol unit derived from bisphenol A and another resin (p) soluble in phenol.

In the depolymerization step, a resin molded body as an example of waste plastic is depolymerized in the presence of phenol to obtain a decomposed liquid (BPA + PHL + p) containing bisphenol A, phenol and another resin. At this time, water, an aliphatic alcohol, or the like can be used as a decomposition agent, and a basic catalyst is preferably used as a catalyst. The decomposed liquid (BPA + PHL + p) is usually subjected to solid-liquid separation (e.g., filtration) to obtain homogeneous solution, and the homogeneous solution is used as an aromatic monoalcohol-containing solution in the step 1.

In the step 1, the aromatic monoalcohol-containing solution containing bisphenol A, phenol, and another resin is first cooled to precipitate a crystalline adduct (BPA-PHL) of bisphenol A and phenol to obtain a slurry of bisphenol A. Then, the slurry containing the precipitated crystalline adduct of bisphenol A and phenol is separated into a crude cake of the crystalline adduct (BPA-PHL) of bisphenol A and phenol and a mother liquor by solid-liquid separation to obtain the crude cake.

In the step 2, the crude cake of the crystalline adduct (BPA-PHL) of bisphenol A and phenol is washed with heated water or a heated aromatic hydrocarbon to obtain a purified cake.

In the adsorption purification step, the purified cake obtained in the step 2 is first dissolved in phenol or melted to obtain a solution in which bisphenol A is dissolved in phenol. Then, the obtained solution is purified by contact with a solid adsorbent to obtain an adsorption-purified liquid containing bisphenol A (PHL solution of BPA).

Further, in the step X, the adsorption-purified liquid containing bisphenol A is supplied to a BPA production plant for continuously producing bisphenol A from phenol and acetone. This makes it possible to purify and recover, in the BPA production plant, bisphenol A in the adsorption-purified liquid together with bisphenol A generated by a condensation reaction between acetone and phenol.

The BPA production plant to which the adsorption-purified liquid is supplied performs a process including the following step A1 to step G1:

step A1: a step in which acetone and phenol are subjected to dehydration condensation in the presence of an acid catalyst to obtain a reaction liquid A1 containing bisphenol A;
step B1: a step in which the unreacted acetone and water are distilled off from the reaction liquid A1 to obtain a concentrated liquid B1;
step C1: a step in which a slurry obtained by subjecting the concentrated liquid B1 to crystallization is separated into a mother liquor C1 and a cake c1 by solid-liquid separation;
step D1: a step in which the cake c1 is purified to obtain the bisphenol A;
step E1: a step in which part of the mother liquor C1 is circulated to be supplied to the step A1;
step F1: a step in which the bisphenol A in the mother liquor C1 is decomposed into phenol and isopropenylphenol under alkaline conditions, and the phenol and the isopropenylphenol are then recombined to generate bisphenol A so that a solution F1 containing the bisphenol A is obtained; and
step G1: a step in which the solution F1 is supplied to the step A1.

Among these, the adsorption-purified liquid containing bisphenol A is preferably supplied to at least one step selected from the group consisting of the step A1, the step B1, the step C1, the step E1, the step F1, and the step G1.

It should be noted that the method for producing a bisphenol of the present invention may be any one of the methods for producing a bisphenol (II) to (V) in which the crude cake obtained in the step 1 is used in the adsorption purification step without performing the step 2.

[Method for Producing Recycled Polycarbonate Resin]

The present invention relates to a method for producing a recycled polycarbonate resin, the method including the steps of: obtaining a bisphenol by the method for producing a bisphenol of the present invention; and producing a recycled polycarbonate resin by using a bisphenol raw material containing the obtained bisphenol (hereinafter sometimes referred to as "method for producing a recycled polycarbonate resin of the present invention").

The recycled polycarbonate resin obtained by the method for producing a recycled polycarbonate resin of the present invention can be produced by subjecting a bisphenol raw material containing a bisphenol (recycled bisphenol) obtained by the method for producing a bisphenol of the present invention and a diester carbonate raw material such as diphenyl carbonate to, for example, a transesterification reaction in the presence of an alkali metal compound and/or an

alkaline-earth metal compound. It should be noted that as a bisphenol raw material, the recycled bisphenol may be used alone or the recycled bisphenol and a bisphenol obtained by a method other than the method for producing a bisphenol of the present invention may be used in combination.

The transesterification reaction can be performed by a publicly-known method appropriately selected, and will be described below with reference to a case where bisphenol A and diphenyl carbonate are used as raw materials.

In the method for producing a recycled polycarbonate resin of the present invention, diphenyl carbonate is preferably used in an excessive amount relative to the amount of bisphenol A. The amount of diphenyl carbonate used relative to the amount of bisphenol A is preferably large from the viewpoint that the produced recycled polycarbonate resin contains few terminal hydroxyl groups and the thermal stability of the polymer is excellent, but is preferably small from the viewpoint that the speed of the transesterification reaction is high and a recycled polycarbonate resin having a desired molecular weight is easy to produce. For these reasons, the amount of diphenyl carbonate used per mole of bisphenol A is usually 1.001 mol or more, preferably 1.002 mol or more and is usually 1.3 mol or less, preferably 1.2 mol or less.

As for a method for supplying the raw materials, bisphenol A and diphenyl carbonate can be supplied as solids, but one or both of these is/are preferably supplied in a liquid state by melting.

When diphenyl carbonate and bisphenol A are subjected to a transesterification reaction to produce a recycled polycarbonate resin, a transesterification catalyst is usually used. In the above-described method for producing a recycled polycarbonate resin, an alkali metal compound and/or an alkaline-earth metal compound are/is preferably used as such a transesterification catalyst. These may be used alone or in combination of two or more of them at any ratio. From a practical viewpoint, an alkali metal compound is preferably used.

The amount of the catalyst used per mole of bisphenol A or diphenyl carbonate is usually 0.05 μmol or more, preferably 0.08 μmol or more, even more preferably 0.10 μmol or more and is usually 100 μmol or less, preferably 50 μmol or less, even more preferably 20 μmol or less. When the amount of the catalyst to be used is within the above range, it is easy to obtain polymerization activity necessary to produce a recycled polycarbonate resin having a desired molecular weight and it is easy to obtain a recycled polycarbonate resin having excellent polymer color hue and excellent fluidity during molding due to prevention of excessive polymer branching.

In order to produce a recycled polycarbonate resin by the above-described method, it is preferred that both the raw materials are continuously supplied to a raw material mixing tank, and the resulting mixture and a transesterification catalyst is continuously supplied to a polymerization tank.

In the production of a recycled polycarbonate resin by transesterification, both the raw materials supplied to the raw material mixing tank are usually stirred uniformly and then supplied to the polymerization tank, to which the catalyst is added, to produce a polymer.

Examples

The present invention will be described below in more detail with reference to examples, but the present invention is not limited to the following examples as long as the gist thereof is not exceeded.

[Raw Materials and Reagents]

- As a polycarbonate resin, a polycarbonate resin "NOVAREX (registered trademark) M7027BF" manufactured by Mitsubishi Chemical Engineering Plastics Co., Ltd. was used.

- As a compound product of a polycarbonate resin and a PET resin (PC/PET resin), "MB2105" manufactured by Mitsubishi Chemical Engineering Plastics Co., Ltd. was used.

- As a compound product of a polycarbonate resin and a PBT resin (PC/PBT resin), "MB4309R" manufactured by Mitsubishi Chemical Engineering Plastics Co., Ltd. was used.

- As a compound product of a polycarbonate resin and an ABS resin (PC/ABS resin), "MB2213R" manufactured by Mitsubishi Chemical Engineering Plastics Co., Ltd. was used.

- As polyethylene terephthalate (PET), polybutylene terephthalate (PBT), an ABS resin (ABS), polyamide 66 (PA66), a phenol resin (PHL resin), an acrylic resin (PMMA), a thermoplastic polyurethane (TPU), and polylactic acid (PLA), their resin pellets for testing manufactured by Standard Test Piece K.K. were used.

- As phenol (PHL), dimethyl carbonate, methanol, sodium hydrogen carbonate, potassium hydroxide, dilute sulfuric

acid, toluene, dodecanethiol, acetonitrile, cesium carbonate, activated carbon (powder), and biphenyl, reagents manufactured by FUJIFILM Wako Pure Chemical Corporation were used.

· As bisphenol A and diphenyl carbonate, products of Mitsubishi Chemical Corporation were used.

[Analysis of Polymers]

Quantification by GPC

· Device: Agilent 1100

PLgel 5 μm 500 Å 300 × 7.5 mm I.D. × 2
PLgel 5 μm 100 Å 300 × 7.5 mm I.D. × 1

· Analysis temperature: 40°C
· Eluant: Tetrahydrofuran
· Analysis time: 30 minutes
· Polymers having a molecular weight of 10000 or more were quantified based on the sum of peaks detected from minute 15.0 to minute 17.0 (in terms of polystyrene standards)

[Analysis of Bisphenol A]

· Device: "LC10A" manufactured by SHIMADZU CORPORATION
Unison UK C18 3 μm 250 × 4.6 mm I.D.
· Analysis temperature: 40°C
· Eluant composition: A 10%/B 90%, Isocratic mode

Liquid A Water
Liquid B Acetonitrile

· Analysis time: 30 minutes

[Analysis of Toluene]

· Device: GC-2014 manufactured by SHIMADZU CORPORATION
Agilent DB-1 0.530 mm × 30 m 1.50 μm
· Method: FID
· Vaporizing chamber temperature: 230°C
· Detector temperature: 300°C
· From minute 0 to minute 5 of analysis time, the temperature of the column was maintained at 50°C, from minute 5 to minute 30 of analysis time, the temperature of the column was gradually increased to 280°C, and from minute 30 to minute 40 of analysis time, the temperature of the column was maintained at 280°C.
· Quantification: Internal standard method using biphenyl as internal standard material

[Measurement of Electric Conductivity of Water Phase in Water Washing Step]

· Device: Electric conductivity meter, COND METER D-71 manufactured by HORIBA, Ltd.

Demineralized water having an electric conductivity of 0.9 μS/cm was used as water supplied in a water washing step.

[Viscosity-Average Molecular Weight]

The specific viscosity (ηsp) at 20°C of a solution obtained by dissolving a polycarbonate resin in methylene chloride (concentration 6.0 g/L) was measured using an Ubbelohde viscometer, and the viscosity-average molecular weight (Mv) of the polycarbonate resin was calculated by the following formula.

$$\eta sp/C = [\eta](1 + 0.28\ \eta sp)$$

$$[\eta] = 1.23 \times 10^{-4}\ Mv^{0.83}$$

[Pellet YI]

Pellet YI (transparency of a polycarbonate resin (PC)) was evaluated by measuring the YI value (yellowness index value) of reflected light from polycarbonate resin pellets in accordance with ASTM D1925. A spectrophotometric colorimeter "CM-5" manufactured by KONICA MINOLTA, INC. was used as a device, and a measurement diameter of 30 mm and an SCE mode were selected as measurement conditions.

Calibration glass for petri dish "CM-A212" was inserted into a measurement part, the measurement part was covered with a zero calibration box "CM-A124" to perform zero calibration, and white calibration was then performed using a built-in white calibration plate. Then, measurement was performed using a white calibration plate "CM-A210" to confirm that L* was 99.40 ± 0.05, a* was 0.03 ± 0.01, b* was -0.43 ± 0.01, YI was -0.58 ± 0.01.

The YI was measured in a state where a cylindrical glass container having an inner diameter of 30 mm and a height of 50 mm was filled with the pellets to a height of about 40 mm. The pellets were taken out of the glass container and the YI was then again measured. This operation was repeated twice, and the average of a total of three measured values was used.

[Example 1]

(Preparation of Homogeneous Solution)

First, 30 g of bisphenol A, 85 g of phenol, 30 g of water, and 5 g of PET pellets were placed in a device equipped with a three-necked glass reaction vessel, an aluminum block thermostatic bath, and a magnetic stirrer (Personal Organic Synthesizer PPV-5460 manufactured by TOKYO RIKAKIKAI CO., LTD.), and the resultant was stirred at 85°C for 2 hours. After a lapse of 2 hours, the resultant was filtered through a glass filter equipped with a vacuum pump to obtain 150.0 g of a filtrate. The filtrate was used as a homogeneous solution A. The homogeneous solution A was analyzed to determine the amount of polymers contained therein. As a result, 3.3% by mass of a component having a molecular weight of 10000 or more was detected, from which it was found that 5.0 g of PET was dissolved in the homogeneous solution A (3.3% by mass × homogeneous solution 150.0 g ÷ 100 = 5.0 g).

(Obtainment of Crude Cake of Bisphenol (Step 1))

The homogeneous solution A was placed in a three-necked glass reaction vessel and cooled until the internal temperature was reduced to 20°C to precipitate a crystal of bisphenol A so that a slurry was obtained. The slurry was subjected to solid-liquid separation using a glass filter equipped with a vacuum pump so that 45 g of a crude cake A of bisphenol A was obtained. The crude cake A was analyzed to determine the amount of polymers contained therein. As a result, 2.0% by mass of a component having a molecular weight of 10000 or more was detected, from which it was found that 0.9 g of PET was contained in the crude cake A (2.0% by mass × crude cake 45 g ÷ 100 = 0.9 g). The removal rate of PET achieved by crude cake obtainment was 82% by mass (100 - 0.9 g/5.0 g × 100 = 82% by mass).

(Obtainment of Purified Cake of Bisphenol (Step 2))

The crude cake A was placed in a 200-mL glass beaker, 120 g of demineralized water heated to 60°C was supplied thereto, and the resultant was stirred with a glass rod to obtain a slurry. The slurry was subjected to solid-liquid separation using a glass filter equipped with a vacuum pump. The same operation was performed again so that 34 g of a purified cake A of bisphenol A was obtained. The purified cake A was analyzed to determine the amount of polymers contained therein. As a result, the amount of a component having a molecular weight of 10000 or more was equal to or less than a detection lower limit of 0.1% by mass. The final removal rate of PET was 99% by mass or more.

[Example 2]

(Preparation of Homogeneous Solution)

In the same manner as in Example 1 except that PET was changed to PBT, 148.7 g of a homogeneous solution B was obtained. Some of the PBT pellets were filtered out by the glass filter. The amount of polymers contained in the homogeneous solution B was measured and, as a result, 2.5% by mass of a component having a molecular weight of 10000 or more was detected, from which it was found that 3.7 g of PBT was dissolved in the homogeneous solution B (2.5% by mass × homogeneous solution 148.7 g ÷ 100 = 3.7 g).

(Obtainment of Crude Cake of Bisphenol (Step 1))

The homogeneous solution B was placed in a three-necked glass reaction vessel and cooled until the internal temperature was reduced to 20°C to precipitate a crystal of bisphenol A so that a slurry was obtained. The slurry was subjected to solid-liquid separation using a glass filter equipped with a vacuum pump so that 43 g of a crude cake B of bisphenol A was obtained. The crude cake B was analyzed to determine the amount of polymers contained therein. As a result, 1.4% by mass of a component having a molecular weight of 10000 or more was detected, from which it was found that 0.6 g of PBT was contained in the crude cake B (1.4% by mass × crude cake 43 g ÷ 100 = 0.6 g). The removal rate of PBT achieved by crude cake obtainment was 84% by mass (100 - 0.6 g/3.7 g × 100 = 84% by mass).

(Obtainment of Purified Cake of Bisphenol (Step 2))

The crude cake B was placed in a 200-mL glass beaker, 120 g of demineralized water heated to 60°C was supplied thereto, and the resultant was stirred with a glass rod to obtain a slurry. The slurry was subjected to solid-liquid separation using a glass filter equipped with a vacuum pump. The same operation was performed again so that 30 g of a purified cake B of bisphenol A was obtained. The purified cake B was analyzed to determine the amount of polymers contained therein. As a result, the amount of a component having a molecular weight of 10000 or more was equal to or less than a detection lower limit of 0.1% by mass. The final removal rate of PBT was 99% by mass or more.

[Example 3]

(Preparation of Homogeneous Solution)

In the same manner as in Example 1 except that PET was changed to an ABS resin, 149.0 g of a homogeneous solution C was obtained. Some of the ABS resin pellets were filtered out by the glass filter. The amount of polymers contained in the homogeneous solution C was measured and, as a result, 2.7% by mass of a component having a molecular weight of 10000 or more was detected, from which it was found that 4.0 g of the ABS resin was dissolved in the homogeneous solution C (2.7% by mass × homogeneous solution 149.0 g ÷ 100 = 4.0 g).

(Obtainment of Crude Cake of Bisphenol (Step 1))

The homogeneous solution C was placed in a three-necked glass reaction vessel and cooled until the internal temperature was reduced to 20°C to precipitate a crystal of bisphenol A so that a slurry was obtained. The slurry was subjected to solid-liquid separation using a glass filter equipped with a vacuum pump so that 44 g of a crude cake C of bisphenol A was obtained. The crude cake C was analyzed to determine the amount of polymers contained therein. As a result, 1.4% by mass of a component having a molecular weight of 10000 or more was detected, from which it was found that 0.6 g of the ABS resin was contained in the crude cake C (1.4% by mass × crude cake 44 g ÷ 100 = 0.6 g). The removal rate of the ABS resin achieved by crude cake obtainment was 85% by mass (100 - 0.6 g/4.0 g × 100 = 85% by mass).

(Obtainment of Purified Cake of Bisphenol (Step 2))

The crude cake C was placed in a 200-mL glass beaker, 120 g of demineralized water at room temperature was supplied thereto, and the resultant was stirred with a glass rod to obtain a slurry. The slurry was subjected to solid-liquid separation using a glass filter equipped with a vacuum pump. The same operation was performed again so that 31 g of a purified cake C of bisphenol A was obtained. The purified cake C was analyzed to determine the amount of polymers contained therein. As a result, a component having a molecular weight of 10000 or more was equal to or less than a detection lower limit of 0.1% by mass. The final removal rate of the ABS resin was 99% by mass or more.

[Example 4]

(Preparation of Homogeneous Solution)

In the same manner as in Example 1 except that PET was changed to PA66, 150.0 g of a homogeneous solution D was obtained. The amount of polymers contained in the homogeneous solution D was measured and, as a result, 3.3% by mass of a component having a molecular weight of 10000 or more was detected, from which it was found that 5.0 g of PA66 was dissolved in the homogeneous solution D (3.3% by mass × homogeneous solution 150.0 g ÷ 100 = 5.0 g).

(Obtainment of Crude Cake of Bisphenol (Step 1))

The homogeneous solution D was placed in a three-necked glass reaction vessel and cooled until the internal temperature was reduced to 20°C to precipitate a crystal of bisphenol A so that a slurry was obtained. The slurry was subjected to solid-liquid separation using a glass filter equipped with a vacuum pump so that 44 g of a crude cake D of bisphenol A was obtained. The crude cake D was analyzed to determine the amount of polymers contained therein. As a result, 1.4% by mass of a component having a molecular weight of 10000 or more was detected, from which it was found that 0.6 g of PA66 was contained in the crude cake D (1.4% by mass × crude cake 44 g ÷ 100 = 0.6 g). The removal rate of PA66 achieved by crude cake obtainment was 88% by mass (100 - 0.6 g/5.0 g × 100 = 88% by mass).

(Obtainment of Purified Cake of Bisphenol (Step 2))

The crude cake D was placed in a 200-mL glass beaker, 120 g of toluene heated to 60°C was supplied thereto, and the resultant was stirred with a glass rod to obtain a slurry. The slurry was subjected to solid-liquid separation using a glass filter equipped with a vacuum pump. The same operation was performed again so that 30 g of a purified cake D of bisphenol A was obtained. The purified cake D was analyzed to determine the amount of polymers contained therein. As a result, the amount of a component having a molecular weight of 10000 or more was equal to or less than a detection lower limit of 0.1% by mass. The final removal rate of PA66 was 99% by mass or more.

[Example 5]

(Preparation of Homogeneous Solution)

In the same manner as in Example 1 except that PET was changed to PMMA, 149.9 g of a homogeneous solution E was obtained. The amount of polymers contained in the homogeneous solution E was measured and, as a result, 3.3% by mass of a component having a molecular weight of 10000 or more was detected, from which it was found that 4.9 g of PMMA was dissolved in the homogeneous solution E (3.3% by mass × homogeneous solution 149.0 g ÷ 100 = 4.9 g).

(Obtainment of crude cake of bisphenol (Step 1))

The homogeneous solution E was placed in a three-necked glass reaction vessel and cooled until the internal temperature was reduced to 20°C to precipitate a crystal of bisphenol A so that a slurry was obtained. The slurry was subjected to solid-liquid separation using a glass filter equipped with a vacuum pump so that 45 g of a crude cake E of bisphenol A was obtained. The crude cake E was analyzed to determine the amount of polymers contained therein. As a result, 2.2% by mass of a component having a molecular weight of 10000 or more was detected, from which it was found that 1.0 g of PMMA was contained in the crude cake E (2.2% by mass × crude cake 45 g ÷ 100 = 1.0 g). The removal rate of PMMA achieved by crude cake obtainment was 80% by mass (100 - 1.0 g/4.9 g × 100 = 80% by mass).

(Obtainment of Purified Cake of Bisphenol (Step 2))

The crude cake E was placed in a 200-mL glass beaker, 120 g of toluene heated to 60°C was supplied thereto, and the resultant was stirred with a glass rod to obtain a slurry. The slurry was subjected to solid-liquid separation using a glass filter equipped with a vacuum pump. The same operation was performed again so that 30 g of a purified cake E of bisphenol A was obtained. The purified cake E was analyzed to determine the amount of polymers contained therein. As a result, the amount of a component having a molecular weight of 10000 or more was equal to or less than a detection lower limit of 0.1% by mass. The final removal rate of PMMA was 99% by mass or more.

[Example 6]

(Preparation of Homogeneous Solution)

In the same manner as in Example 1 except that PET was changed to TPU, 148.9 g of a homogeneous solution F was obtained. Some of the TPU pellets were filtered out by the glass filter. The amount of polymers contained in the homogeneous solution F was measured and, as a result, 2.6% by mass of a component having a molecular weight of 10000 or more was detected, from which it was found that 3.9 g of TPU was dissolved in the homogeneous solution F (2.6% by mass × homogeneous solution 148.9 g ÷ 100 = 3.9 g).

(Obtainment of Crude Cake of Bisphenol (Step 1))

The homogeneous solution F was placed in a three-necked glass reaction vessel and cooled until the internal temperature was reduced to 20°C to precipitate a crystal of bisphenol A so that a slurry was obtained. The slurry was subjected to solid-liquid separation using a glass filter equipped with a vacuum pump so that 43 g of a crude cake F of bisphenol A was obtained. The crude cake F was analyzed to determine the amount of polymers contained therein. As a result, 1.5% by mass of a component having a molecular weight of 10000 or more was detected, from which it was found that 0.6 g of TPU was contained in the crude cake F (1.5% by mass × crude cake 43 g ÷ 100 = 0.6 g). The removal rate of TPU achieved by crude cake obtainment was 85% by mass (100 - 0.6 g/3.9 g × 100 = 85% by mass).

(Obtainment of Purified Cake of Bisphenol (Step 2))

The crude cake F was placed in a 200-mL glass beaker, 120 g of demineralized water heated to 60°C was supplied thereto, and the resultant was stirred with a glass rod to obtain a slurry. The slurry was subjected to solid-liquid separation using a glass filter equipped with a vacuum pump. The same operation was performed again so that 32 g of a purified cake F of bisphenol A was obtained. The purified cake F was analyzed to determine the amount of polymers contained therein. As a result, the amount of a component having a molecular weight of 10000 or more was equal to or less than a detection lower limit of 0.1% by mass. The final removal rate of TPU was 99% by mass or more.

[Example 7]

(Preparation of Homogeneous Solution)

In the same manner as in Example 1 except that PET was changed to PLA, 148.8 g of a homogeneous solution G was obtained. Some of the PLA pellets were filtered out by the glass filter. The amount of polymers contained in the homogeneous solution G was measured and, as a result, 2.5% by mass of a component having a molecular weight of 10000 or more was detected, from which it was found that 3.7 g of PLA was dissolved in the homogeneous solution G (2.5% by mass × homogeneous solution 148.8 g ÷ 100 = 3.7 g).

(Obtainment of Crude Cake of Bisphenol (Step 1))

The homogeneous solution G was placed in a three-necked glass reaction vessel and cooled until the internal temperature was reduced to 20°C to precipitate a crystal of bisphenol A so that a slurry was obtained. The slurry was subjected to solid-liquid separation using a glass filter equipped with a vacuum pump so that 42 g of a crude cake G of bisphenol A was obtained. The crude cake G was analyzed to determine the amount of polymers contained therein. As a result, 1.1% by mass of a component having a molecular weight of 10000 or more was detected, from which it was found that 0.5 g of PLA was contained in the crude cake G (1.1% by mass × crude cake 42 g ÷ 100 = 0.5 g). The removal rate of PLA achieved by crude cake obtainment was 86% by mass (100 - 0.5 g/3.7 g × 100 = 86% by mass).

(Obtainment of Purified Cake of Bisphenol (Step 2))

The crude cake G was placed in a 200-mL glass beaker, 120 g of toluene at room temperature was supplied thereto, and the resultant was stirred with a glass rod to obtain a slurry. The slurry was subjected to solid-liquid separation using a glass filter equipped with a vacuum pump. The same operation was performed again so that 31 g of a purified cake G of bisphenol A was obtained. The purified cake G was analyzed to determine the amount of polymers contained therein. As a result, a component having a molecular weight of 10000 or more was equal to or less than a detection lower limit of 0.1% by mass. The final removal rate of PLA was 99% by mass or more.

[Example 8]

(Preparation of Homogeneous Solution)

In the same manner as in Example 1 except that PET was changed to a phenol resin (PHL resin), 150.0 g of a homogeneous solution H was obtained. The amount of polymers contained in the homogeneous solution H was measured and, as a result, 3.3% by mass of a component having a molecular weight of 10000 or more was detected, from which it was found that 5.0 g of the phenol resin was dissolved in the homogeneous solution H (3.3% by mass × homogeneous solution 150.0 g ÷ 100 = 5.0 g).

(Obtainment of Crude Cake of Bisphenol (Step 1))

The homogeneous solution H was placed in a three-necked glass reaction vessel and cooled until the internal temperature was reduced to 20°C to precipitate a crystal of bisphenol A so that a slurry was obtained. The slurry was subjected to solid-liquid separation using a glass filter equipped with a vacuum pump so that 42 g of a crude cake H of bisphenol A was obtained. The crude cake H was analyzed to determine the amount of polymers contained therein. As a result, 1.3% by mass of a component having a molecular weight of 10000 or more was detected, from which it was found that 0.5 g of the phenol resin was contained in the crude cake H (1.3% by mass × crude cake 42 g ÷ 100 = 0.5 g). The removal rate of the phenol resin achieved by crude cake obtainment was 90% by mass (100 - 0.5 g/5.0 g × 100 = 90% by mass).

(Obtainment of Purified Cake of Bisphenol (Step 2))

The crude cake H was placed in a 200-mL glass beaker, 120 g of demineralized water heated to 60°C was supplied thereto, and the resultant was stirred with a glass rod to obtain a slurry. The slurry was subjected to solid-liquid separation using a glass filter equipped with a vacuum pump. The same operation was performed again so that 30 g of a purified cake H of bisphenol A was obtained. The purified cake H was analyzed to determine the amount of polymers contained therein. As a result, a component having a molecular weight of 10000 or more was equal to or less than a detection lower limit of 0.1% by mass. The final removal rate of the phenol resin was 99% by mass or more.

[Comparative Example 1]

(Preparation of Homogeneous Solution)

First, 50 g of bisphenol A, 40 g of dimethyl carbonate, and 10 g of ABS resin pellets were placed in a device equipped with a three-necked glass reaction vessel, an aluminum block thermostatic bath, and a magnetic stirrer (Personal Organic Synthesizer PPV-5460 manufactured by TOKYO RIKAKIKAI CO., LTD.), and the resultant was stirred at 85°C for 2 hours. After a lapse of 2 hours, the resultant was filtered through a glass filter equipped with a vacuum pump so that 98.6 g of a filtrate was obtained. The filtrate was used as a homogeneous solution I. The homogeneous solution I was analyzed to determine the amount of polymers contained therein. As a result, 8.6% by mass of a component having a molecular weight of 10000 or more was detected, from which it was found that 8.5 g of the ABS resin was dissolved in the homogeneous solution I (8.6% by mass × homogeneous solution 98.6 g ÷ 100 = 8.5 g).

(Obtainment of Crude Cake of Bisphenol)

The homogeneous solution I was placed in a three-necked glass reaction vessel and cooled until the internal temperature was reduced to 50°C. After the internal temperature was confirmed to be reduced to 50°C by cooling, 80 g of toluene was supplied and the resultant was further cooled to 20°C to precipitate a crystal of bisphenol A so that a slurry was obtained. The slurry was subjected to solid-liquid separation using a glass filter equipped with a vacuum pump so that 46 g of a crude cake I of bisphenol A was obtained. The crude cake I was analyzed to determine the amount of polymers contained therein. As a result, 15.9% by mass of a component having a molecular weight of 10000 or more was detected, from which it was found that 7.3 g of the ABS resin was contained in the crude cake I (15.9% by mass × crude cake 46 g ÷ 100 = 7.3 g). The removal rate of the ABS resin achieved by crude cake obtainment was 14% by mass (100 - 7.3 g/8.5 g × 100 = 14% by mass).

(Obtainment of Purified Cake of Bisphenol)

The crude cake I was placed in a 200-mL glass beaker, 150 g of toluene heated to 60°C was supplied thereto, and the resultant was stirred with a glass rod to obtain a slurry. The slurry was subjected to solid-liquid separation using a glass filter equipped with a vacuum pump. The same operation was performed again so that 44 g of a purified cake I of bisphenol

A was obtained. The purified cake I was analyzed to determine the amount of polymers contained therein. As a result, 15.5% by mass of a component having a molecular weight of 10000 or more was detected, from which it was found that 6.8 g of the ABS resin was contained in the purified cake I (15.5% by mass × purified cake 44 g ÷ 100 = 6.8 g). The final removal rate of the ABS resin was 20% by mass (100 - 6.8 g/8.5 g × 100 = 20% by mass).

[Comparative Example 2]

(Preparation of Homogeneous Solution)

In the same manner as in Comparative Example 1 except that the ABS resin was changed to PMMA, 100.0 g of a homogeneous solution J was obtained. The homogeneous solution J was analyzed to determine the amount of polymers contained therein. As a result, 10.0% by mass of a component having a molecular weight of 10000 or more was detected, from which it was found that 10.0 g of PMMA was dissolved in the homogeneous solution J (10.0% by mass × homogeneous solution 100.0 g ÷ 100 = 10.0 g).

(Obtainment of Crude Cake of Bisphenol)

The homogeneous solution J was placed in a three-necked glass reaction vessel and cooled until the internal temperature was reduced to 50°C. After the internal temperature was confirmed to be reduced to 50°C by cooling, 80 g of toluene was supplied and the resultant was further cooled to 20°C to precipitate a crystal of bisphenol A so that a slurry was obtained. The slurry was subjected to solid-liquid separation using a glass filter equipped with a vacuum pump so that 50 g of a crude cake J of bisphenol A was obtained. The crude cake J was analyzed to determine the amount of polymers contained therein. As a result, 19.7% by mass of a component having a molecular weight of 10000 or more was detected, from which it was found that 9.9 g of PMMA was contained in the crude cake J (19.7% by mass × crude cake 50 g ÷ 100 = 9.9 g). The removal rate of PMMA achieved by crude cake obtainment was 1% by mass (100 - 9.9 g/10.0 g × 100 = 1% by mass).

(Obtainment of Purified Cake of Bisphenol)

The crude cake J was placed in a 200-mL glass beaker, 150 g of toluene heated to 60°C was supplied thereto, and the resultant was stirred with a glass rod to obtain a slurry. The slurry was subjected to solid-liquid separation using a glass filter equipped with a vacuum pump. The same operation was performed again so that 45 g of a purified cake J of bisphenol A was obtained. The purified cake J was analyzed to determine the amount of polymers contained therein. As a result, 21.0% by mass of a component having a molecular weight of 10000 or more was detected, from which it was found that 9.5 g of PMMA was contained in the purified cake J (21.0% by mass × purified cake 45 g ÷ 100 = 9.5 g). The final removal rate of PMMA was 5% by mass (100 - 9.5 g/10.0 g × 100 = 5% by mass).

[Comparative Example 3]

(Preparation of Homogeneous Solution)

In the same manner as in Comparative Example 1 except that the ABS resin was changed to PLA, 99.6 g of a homogeneous solution K was obtained. The homogeneous solution K was analyzed to determine the amount of polymers contained therein. As a result, 9.6% by mass of a component having a molecular weight of 10000 or more was detected, from which it was found that 9.6 g of PLA was dissolved in the homogeneous solution K (9.6% by mass × homogeneous solution 99.6 g ÷ 100 = 9.6 g).

(Obtainment of Crude Cake of Bisphenol)

The homogeneous solution K was placed in a three-necked glass reaction vessel and cooled until the internal temperature was reduced to 50°C. After the internal temperature was confirmed to be reduced to 50°C by cooling, 80 g of toluene was supplied and the resultant was further cooled to 20°C to precipitate a crystal of bisphenol A so that a slurry was obtained. The slurry was subjected to solid-liquid separation using a glass filter equipped with a vacuum pump so that 48 g of a crude cake K of bisphenol A was obtained. The crude cake K was analyzed to determine the amount of polymers contained therein. As a result, 15.3% by mass of a component having a molecular weight of 10000 or more was detected, from which it was found that 7.3 g of PLA was contained in the crude cake K (15.3% by mass × crude cake 48 g ÷ 100 = 7.3 g). The removal rate of PLA achieved by crude cake obtainment was 24% by mass (100 - 7.3 g/9.6 g × 100 = 24% by mass).

(Obtainment of Purified Cake of Bisphenol)

The crude cake K was placed in a 200-mL glass beaker, 150 g of toluene heated to 60°C was supplied thereto, and the resultant was stirred with a glass rod to obtain a slurry. The slurry was subjected to solid-liquid separation using a glass filter equipped with a vacuum pump. The same operation was performed again so that 43 g of a purified cake K of bisphenol A was obtained. The purified cake K was analyzed to determine the amount of polymers contained therein. As a result, 15.7% by mass of a component having a molecular weight of 10000 or more was detected, from which it was found that 6.8 g of PLA was contained in the purified cake K (15.7% by mass × purified cake 43 g ÷ 100 = 6.8 g). The final removal rate of PLA was 29% by mass (100 - 6.8 g/9.6 g × 100 = 29% by mass).

[Comparative Example 4]

(Preparation of Homogeneous Solution)

In the same manner as in Comparative Example 1 except that the ABS resin was changed to a phenol resin, 99.8 g of a homogeneous solution L was obtained. The homogeneous solution L was analyzed to determine the amount of polymers contained therein. As a result, 9.8% by mass of a component having a molecular weight of 10000 or more was detected, from which it was found that 9.8 g of the phenol resin was dissolved in the homogeneous solution L (9.8% by mass × homogeneous solution 99.8 g ÷ 100 = 9.8 g).

(Obtainment of Crude Cake of Bisphenol)

The homogeneous solution L was placed in a three-necked glass reaction vessel and cooled until the internal temperature was reduced to 50°C. After the internal temperature was confirmed to be reduced to 50°C by cooling, 80 g of toluene was supplied and the resultant was further cooled to 20°C to precipitate a crystal of bisphenol A so that a slurry was obtained. The slurry was subjected to solid-liquid separation using a glass filter equipped with a vacuum pump so that 49 g of a crude cake L of bisphenol A was obtained. The crude cake L was analyzed to determine the amount of polymers contained therein. As a result, 17.9% by mass of a component having a molecular weight of 10000 or more was detected, from which it was found that 8.8 g of the phenol resin was contained in the crude cake L (17.9% by mass × crude cake 49 g ÷ 100 = 8.8 g). The removal rate of the phenol resin achieved by crude cake obtainment was 10% by mass (100 - 8.8 g/9.8 g × 100 = 10% by mass).

(Obtainment of Purified Cake of Bisphenol)

The crude cake L was placed in a 200-mL glass beaker, 150 g of toluene heated to 60°C was supplied thereto, and the resultant was stirred with a glass rod to obtain a slurry. The slurry was subjected to solid-liquid separation using a glass filter equipped with a vacuum pump. The same operation was performed again so that 45 g of a purified cake L of bisphenol A was obtained. The purified cake L was analyzed to determine the amount of polymers contained therein. As a result, 16.9% by mass of a component having a molecular weight of 10000 or more was detected, from which it was found that 7.6 g of the phenol resin was contained in the purified cake L (16.9% by mass × purified cake 45 g ÷ 100 = 7.6 g). The final removal rate of the phenol resin was 22% by mass (100 - 7.6 g/9.8 g × 100 = 22% by mass).

[Comparative Example 5]

(Preparation of Homogeneous Solution)

In the same manner as in Comparative Example 1 except that the ABS resin was changed to PMMA and dimethyl carbonate was changed to methylene chloride, 100.0 g of a homogeneous solution M was obtained. The homogeneous solution M was analyzed to determine the amount of polymers contained therein. As a result, 10.0% by mass of a component having a molecular weight of 10000 or more was detected, from which it was found that 10.0 g of PMMA was dissolved in the homogeneous solution M (10.0% by mass × homogeneous solution 100.0 g ÷ 100 = 10.0 g).

(Obtainment of Crude Cake of Bisphenol)

The homogeneous solution M was placed in a three-necked glass reaction vessel and cooled until the internal temperature was reduced to 50°C. After the internal temperature was confirmed to be reduced to 50°C by cooling, 90 g of toluene was supplied and the resultant was further cooled to 20°C to precipitate a crystal of bisphenol A so that a slurry was obtained. The slurry was subjected to solid-liquid separation using a glass filter equipped with a vacuum pump so that 47 g

of a crude cake M of bisphenol A was obtained. The crude cake M was analyzed to determine the amount of polymers contained therein. As a result, 20.1% by mass of a component having a molecular weight of 10000 or more was detected, from which it was found that 9.4 g of PMMA was contained in the crude cake M (20.1% by mass × crude cake 47 g ÷ 100 = 9.4 g). The removal rate of PMMA achieved by crude cake obtainment was 6% by mass (100 - 9.4 g/10.0 g × 100 = 6% by mass).

(Obtainment of Purified Cake of Bisphenol)

The crude cake M was placed in a 200-mL glass beaker, 150 g of toluene heated to 60°C was supplied thereto, and the resultant was stirred with a glass rod to obtain a slurry. The slurry was subjected to solid-liquid separation using a glass filter equipped with a vacuum pump. The same operation was performed again so that 42 g of a purified cake M of bisphenol A was obtained. The purified cake M was analyzed to determine the amount of polymers contained therein. As a result, 20.8% by mass of a component having a molecular weight of 10000 or more was detected, from which it was found that 8.7 g of PMMA was contained in the purified cake M (20.8% by mass × purified cake 42 g ÷ 100 = 8.7 g). The final removal rate of PMMA was 13% by mass (100 - 8.7 g/10.0 g × 100 = 13% by mass).

The results of Examples 1 to 8 and Comparative Examples 1 to 5 are summarized in Table 1.

[Table 1]

| | Type of polymer | Homogeneous solution | Crude cake | Purified cake |
|---|---|---|---|---|
| | | PHL/BPA (mol/mol) | Removal rate (%) | Removal rate (%) |
| Example 1 | PET | 7 | 82 | 99≤ |
| Example 2 | PBT | 7 | 84 | 99≤ |
| Example 3 | ABS | 7 | 85 | 99≤ |
| Example 4 | PA66 | 7 | 88 | 99≤ |
| Example 5 | PMMA | 7 | 80 | 99≤ |
| Example 6 | TPU | 7 | 85 | 99≤ |
| Example 7 | PLA | 7 | 86 | 99≤ |
| Example 8 | PHL resin | 7 | 90 | 99≤ |
| Comparative Example 1 | ABS | 0 | 14 | 20 |
| Comparative Example 2 | PMMA | 0 | 1 | 5 |
| Comparative Example 3 | PLA | 0 | 24 | 20 |
| Comparative Example 4 | PHL resin | 0 | 10 | 22 |
| Comparative Example 5 | PMMA | 0 | 6 | 13 |

[Example 9]

(Depolymerization Step)

In a jacket-type separable flask equipped with a Dimroth condenser, a stirring blade, and a thermometer, 120 g of a polycarbonate resin (120 g ÷ 254 g/mol = 0.472 mol, repeating unit of polycarbonate resin: 254 g/mol), 107 g of an aqueous sodium hydrogen carbonate solution adjusted to 7% by mass, 360 g of phenol, 2.4 g of dodecanethiol, and 12 g of PMMA pellets were placed at room temperature in a nitrogen atmosphere. The resulting reaction liquid was in the form of a slurry.

Then, the internal temperature was increased to 90°C, and the reaction liquid was subjected to reaction for 4 hours while the internal temperature was maintained at 90°C to obtain a decomposed liquid (homogeneous solution). Part of this decomposed liquid was subjected to composition analysis by high-performance liquid chromatography and, as a result, generation of bisphenol A was confirmed.

This decomposed liquid was filtered through a glass filter equipped with a vacuum pump so that 580 g of a filtrate was obtained as a homogeneous solution N. Part of this homogeneous solution N was subjected to composition analysis by GPC. As a result, 2.0% by mass of a component having a molecular weight of 10000 or more was detected, from which it was found that 11.6 g of PMMA was contained in the homogeneous solution N (2.0% by mass × homogeneous solution N

580 g ÷ 100 = 11.6 g).

(Step 1)

The obtained homogeneous solution N was gradually cooled from 90°C to 10°C to obtain a slurry. The obtained slurry was filtered so that 206 g of a crude cake N (solid component) and 355 g of a crude crystallization mother liquor were obtained. The crude cake M was analyzed to determine the amount of polymers contained therein. As a result, 1.4% by mass of a component having a molecular weight of 10000 or more was detected, from which it was found that 2.9 g of PMMA was contained in the crude cake N (1.4% by mass × crude cake 206 g ÷ 100 = 2.9 g). The removal rate of PMMA achieved by crude cake obtainment was 75% by mass (100 - 2.9 g/11.6 g × 100 = 75% by mass).

(Step 2)

450 g of demineralized water heated to 60°C was sprinkled on the crude cake N to wash the crude cake so that 136 g of a purified cake N was obtained. The purified cake N was analyzed to determine the amount of polymers contained therein. As a result, the amount of a component having a molecular weight of 10000 or more was equal to or less than a detection lower limit (0.1% by mass or less). The removal rate of PMMA was 99% by mass or more. It should be noted that as a result of composition analysis of part of the crude cake after washing by high-performance liquid chromatography, it was confirmed that the crude cake after washing was composed of a crystalline adduct that was a phenol adduct of bisphenol A.

(Adsorption Crystallization Step)

The obtained purified cake N was returned into the jacket-type separable flask equipped with a Dimroth condenser, a stirring blade, and a thermometer, 600 g of toluene was added thereto, and the resultant was heated to 75°C to melt the purified cake N so that an organic phase N1 containing bisphenol A (bisphenol solution) was obtained.

This organic phase N1 was subjected to suction filtration by supplying it little by little to a glass filter loaded with 20 g of activated carbon to obtain an organic phase N2 (adsorption-purified liquid). Filterability was excellent.

(Water Washing Step)

The obtained organic phase N2 was again returned into the separable flask, 100 g of water was added thereto, the resultant was stirred for 15 minutes while being maintained at 75°C, and the resultant was then allowed to stand for 10 minutes to separate it into two phases so that an organic phase and a water phase were obtained. This operation was repeated three times to obtain an organic phase N3. The water phase obtained by the fourth operation was discharged into a heatproof jar and cooled to 25°C, and then the electric conductivity thereof was measured and found to be 2.2 μS/cm.

(Purifying Crystallization Step)

Then, the organic phase N3 was cooled to 10°C to obtain a slurry. The obtained slurry was filtered so that 75 g of a cake was obtained.

The obtained cake was dried using a rotary evaporator while being maintained at 85°C for 5 hours so that 60 g of bisphenol A was obtained.

The yield was 56 mol% (60 g ÷ 228 g/mol ÷ 0.472 × 100 = 56 mol%). The obtained bisphenol A was analyzed by gas chromatography and, as a result, the content of toluene (TOL content) was 500 ppm by mass.

[Example 10]

(Depolymerization Step)

In a jacket-type separable flask equipped with a Dimroth condenser, a stirring blade, and a thermometer, 125 g of methanol, 300 g of dimethyl carbonate, and 3 g of potassium hydroxide were placed, and then 200 g of a polycarbonate resin (Number of moles of a repeating unit derived from bisphenol = 200 g ÷ 254 g/mol = 0.787 mol, Molecular weight of bisphenol-derived repeating unit of polycarbonate resin: 254 g/mol) and 20 g of PMMA pellets were placed at room temperature in a nitrogen atmosphere.

Then, the jacket temperature was increased to 80°C. A reaction liquid at the time when the jacket temperature reached 80°C was in the form of a slurry in which part of the polycarbonate resin was undissolved. Further, methanol was refluxed. In such a state, the reaction liquid was subjected to reaction for 3 hours while the jacket temperature was

maintained at 80°C to obtain a decomposed liquid (homogeneous solution).

Part of this decomposed liquid was subjected to composition analysis by high-performance liquid chromatography. As a result, it was confirmed that 24.3% by mass (153 g) of bisphenol A was contained (24.3% by mass × decomposed liquid 628 g ÷ 100 = 153 g).

Dilute sulfuric acid was added to the obtained decomposed liquid until the pH of a water phase became 6, and the resultant was then filtered through a glass filter equipped with a vacuum pump so that 645 g of a homogeneous liquid was obtained. Part of this homogeneous liquid was subjected to composition analysis by GPC. As a result, 3.0% by mass of a component having a molecular weight of 10000 or more was detected, from which it was found that 19.4 g of PMMA was contained in the homogeneous liquid (3.0% by mass × homogeneous liquid 645 g ÷ 100 = 19.4 g).

The homogeneous liquid was transferred into a distillator equipped with a thermometer, a stirring blade, a distillate outlet tube, and a pressure controller, 60 g of phenol was added thereto, the pressure was then reduced from ordinary pressure (760 Torr) to 120 Torr, and the internal temperature was increased from room temperature to 80°C while the amount of a distillate was checked so that 410 g of a distillate fraction was discharged to obtain a still residue.

(Step 1)

To the obtained still residue, 300 g of toluene was added to obtain a homogeneous solution O. This homogeneous solution O was maintained at 80°C and was then gradually cooled to 5°C to obtain a slurry. The obtained slurry was separated into 252 g of a crude cake O and 280 g of a crude crystallization mother liquor by filtration using a centrifugal separator. This crude cake O was analyzed to determine the amount of polymers contained therein. As a result, 1.8% by mass of a component having a molecular weight of 10000 or more was detected, from which it was found that 4.5 g of PMMA was contained in the crude cake O (1.8% by mass × crude cake 252 g ÷ 100 = 4.5 g). The removal rate of PMMA achieved by crude cake obtainment was 77% by mass (100 - 4.5 g/19.4 g × 100 = 77% by mass).

(Step 2)

600 g of toluene heated to 60°C was sprinkled on the crude cake O to wash the crude cake so that 226 g of a purified cake O was obtained. This purified cake O was analyzed to determine the amount of polymers contained therein. As a result, the amount of a component having a molecular weight of 10000 or more was equal to or less than a detection lower limit (0.1% by mass or less). The removal rate of PMMA was 99% by mass or more. It should be noted that as a result of composition analysis of part of the crude cake after washing by high-performance liquid chromatography, it was confirmed that the crude cake after washing was composed of a crystalline adduct that was a phenol adduct of bisphenol A.

(Adsorption Purification Step)

The obtained purified cake O was returned into the jacket-type separable flask equipped with a Dimroth condenser, a stirring blade, and a thermometer, 600 g of toluene and 80 g of water were added thereto, and the resultant was heated to 80°C to melt the purified cake O so that an organic phase O1 containing bisphenol A (bisphenol solution) was obtained.

This organic phase O1 was subjected to suction filtration by supplying it little by little to a glass filter loaded with 20 g of activated carbon to obtain an organic phase O2 (adsorption-purified liquid). Filterability was excellent.

(Water Washing Step)

The obtained organic phase O2 was again returned into the separable flask, 120 g of water was added thereto, the resultant was stirred for 15 minutes while being maintained at 75°C, and the resultant was then allowed to stand for 10 minutes to separate it into two phases so that an organic phase and a water phase were obtained. This operation was repeated three times to obtain an organic phase O3. The water phase obtained by the fourth operation was discharged into a heatproof jar and cooled to 25°C, and then the electric conductivity thereof was measured and found to be 1.8 μS/cm.

(Purifying Crystallization Step)

Then, the organic phase O3 was cooled to 10°C to obtain a slurry. The obtained slurry was filtered so that 134 g of a cake was obtained.

The obtained cake was dried using a rotary evaporator while being maintained at 85°C for 5 hours so that 107 g of bisphenol A was obtained.

The yield was 60 mol% (107 g ÷ 228 g/mol ÷ 0.787 × 100 = 60 mol%). The obtained bisphenol A was analyzed by gas chromatography and, as a result, the content of toluene was 500 ppm by mass.

[Comparative Example 6]

(Depolymerization Step)

The depolymerization step was performed under the same conditions as in Example 10, and generation of bisphenol A was confirmed.

Dilute sulfuric acid was added to the obtained decomposed liquid until the pH of a water phase became 6, and the resultant was then filtered through a glass filter equipped with a vacuum pump so that 645 g of a homogeneous liquid was obtained. Part of this homogeneous liquid was subjected to composition analysis by GPC. As a result, 3.0% by mass of a component having a molecular weight of 10000 or more was detected, from which it was found that 19.4 g of PMMA was contained in the homogeneous liquid (3.0% by mass × homogeneous liquid 645 g ÷ 100 = 19.4 g).

The homogeneous liquid was transferred into a distillator equipped with a thermometer, a stirring blade, a distillate outlet tube, and a pressure controller, the pressure was reduced from ordinary pressure (760 Torr) to 120 Torr, and the internal temperature was increased from room temperature to 80°C while the amount of a distillate was checked so that 290 g of a distillate fraction was discharged to obtain a still residue.

(Step 1)

To the obtained still residue, 360 g of toluene was added to obtain a homogeneous solution P. This homogeneous solution P was maintained at 80°C and was then gradually cooled to 5°C to obtain a slurry. The obtained slurry was separated into 162 g of a crude cake P and 557 g of a crude crystallization mother liquor by filtration using a centrifugal separator. The crude cake P was analyzed to determine the amount of polymers contained therein. As a result, 11.7% by mass of a component having a molecular weight of 10000 or more was detected, from which it was found that 19.0 g of PMMA was contained in the crude cake P (11.7% by mass × crude cake 162 g ÷ 100 = 19.0 g). The removal rate of PMMA achieved by crude cake obtainment was 2% by mass (100 - 19.0 g/19.4 g × 100 = 2% by mass).

(Step 2)

450 g of toluene heated to 60°C was sprinkled on the crude cake P to wash the crude cake so that 158 g of a purified cake P was obtained. This purified cake P was analyzed to determine the amount of polymers contained therein. As a result, 11.6% by mass of a component having a molecular weight of 10000 or more was detected, from which it was found that 18.3 g of PMMA was contained in the crude cake P (11.6% by mass × crude cake 158 g ÷ 100 = 18.3 g). The removal rate of PMMA achieved by purified cake obtainment was 6% by mass (100 - 18.3 g/19.4 g × 100 = 6% by mass).

(Adsorption Purification Step)

The obtained purified cake P was returned into the jacket-type separable flask equipped with a Dimroth condenser, a stirring blade, and a thermometer, 600 g of toluene was added thereto, and the resultant was heated to 80°C to melt the purified cake P so that an organic phase P1 containing bisphenol A (bisphenol solution) was obtained.

This organic phase P1 was subjected to suction filtration by supplying it little by little to a glass filter loaded with 20 g of activated carbon to obtain an organic phase P2 (adsorption-purified liquid). At this time, a filtration speed was reduced from the middle of the filtration, and therefore the time required to purify all the organic phase P1 by adsorption was about three times the time required therefor in Example 9 or 10.

(Water Washing Step)

The obtained organic phase P2 was again returned into the separable flask, 120 g of water was added thereto, the resultant was stirred for 15 minutes while being maintained at 75°C, and the resultant was then allowed to stand for 10 minutes to separate it into two phases so that an organic phase and a water phase were obtained. This operation was repeated three times to obtain an organic phase P3. The water phase obtained by the fourth operation was discharged into a heatproof jar and cooled to 25°C, and then the electric conductivity thereof was measured and found to be 5.2 μS/cm.

(Purifying Crystallization Step)

Then, the organic phase P3 was cooled to 10°C to obtain a slurry. The obtained slurry was filtered so that 155 g of a cake was obtained.

The obtained cake was dried using a rotary evaporator while being maintained at 85°C for 5 hours, but the bisphenol A remained as a cake.

This bisphenol A after drying was analyzed by gas chromatography and, as a result, the content of toluene was 18% by mass.

[Table 2]

| | Type of polymer | Homogeneous solution | Crude cake | Purified cake | BPA |
|---|---|---|---|---|---|
| | | PHL/BPA (mol/mol) | Removal rate (%) | Removal rate (%) | TOL content |
| Example 9 | PMMA | 7.5 | 75 | 99≤ | 500 wtppm |
| Example 10 | PMMA | 1 | 77 | 99≤ | 500 wtppm |
| Comparative Example 6 | PMMA | 0 | 2 | 6 | 18 wt% |

[Example 11]

(Depolymerization Step)

In a jacket-type separable flask equipped with a Dimroth condenser, a stirring blade, and a thermometer, 120 g of a polycarbonate resin (120 g ÷ 254 g/mol = 0.472 mol, Molecular weight of repeating unit of polycarbonate resin: 254 g/mol), 107 g of an aqueous sodium hydrogen carbonate solution adjusted to 7% by mass, 360 g of phenol, 2.4 g of dodecanethiol, and 12 g of PC/ABS alloy pellets (PC content 70% by mass: 1.2 g × 70% by mass ÷ 100 ÷ 254 g/mol = 0.003 mol) were placed at room temperature in a nitrogen atmosphere. The resulting reaction liquid was in the form of a slurry.

Then, the internal temperature was increased to 90°C, and the reaction liquid was subjected to reaction for 4 hours while the internal temperature was maintained at 90°C to obtain a decomposed liquid. The decomposed liquid was cloudy and was not a homogeneous solution.

This decomposed liquid was filtered through a glass filter equipped with a vacuum pump so that 575 g of a filtrate was obtained as a homogeneous solution Q. Part of this homogeneous solution Q was subjected to composition analysis by high-performance liquid chromatography. As a result, generation of bisphenol A was confirmed.

(Step 1)

The obtained homogeneous solution Q was gradually cooled from 90°C to 10°C to obtain a slurry. The obtained slurry was filtered so that 187 g of a crude cake Q (solid component) and 345 g of a crude crystallization mother liquor were obtained.

(Step 2)

450 g of demineralized water heated to 60°C was sprinkled on the crude cake Q to wash the crude cake so that 131 g of a purified cake Q was obtained. Part of this purified cake Q was subjected to composition analysis by high-performance liquid chromatography and, as a result, it was confirmed that the purified cake Q was composed of a crystalline adduct that was a phenol adduct of bisphenol A.

(Adsorption Crystallization Step)

The obtained purified cake Q was returned into the jacket-type separable flask equipped with a Dimroth condenser, a stirring blade, and a thermometer, 600 g of toluene was added thereto, and the resultant was heated to 75°C to melt the purified cake Q so that an organic phase Q1 containing bisphenol A (bisphenol solution) was obtained.

This organic phase Q1 was subjected to suction filtration by supplying it little by little to a glass filter loaded with 20 g of activated carbon to obtain an organic phase Q2 (adsorption-purified liquid). Filterability was excellent.

(Water Washing Step)

The obtained organic phase Q2 was again returned into the separable flask, 100 g of water was added thereto, the resultant was stirred for 15 minutes while being maintained at 75°C, and the resultant was then allowed to stand for 10 minutes to separate it into two phases so that an organic phase and a water phase were obtained. This operation was

repeated three times to obtain an organic phase Q3. The water phase obtained by the fourth operation was discharged into a heatproof jar and cooled to 25°C, and then the electric conductivity thereof was measured and found to be 1.5 μS/cm.

(Purifying Crystallization Step)

Then, the organic phase Q3 was cooled to 10°C to obtain a slurry. The obtained slurry was filtered so that 72 g of a cake was obtained.
The obtained cake was dried using a rotary evaporator while being maintained at 85°C for 5 hours so that 58 g of bisphenol A was obtained.
The yield was 54 mol% (58 g ÷ 228 g/mol ÷ 0.475 mol × 100 = 54 mol%). The obtained bisphenol A was analyzed by gas chromatography and, as a result, the content of toluene was 500 ppm by mass. It should be noted that as a result of analysis of the bisphenol A after drying (product BPA) to determine the amount of polymers contained therein, the amount of a component having a molecular weight of 10000 or more was equal to or less than a detection lower limit (0.1% by mass or less).

[Example 12]

(Depolymerization Step)

In a jacket-type separable flask equipped with a Dimroth condenser, a stirring blade, and a thermometer, 120 g of a polycarbonate resin (120 g ÷ 254 g/mol = 0.472 mol, Molecular weight of repeating unit of polycarbonate resin: 254 g/mol), 107 g of an aqueous sodium hydrogen carbonate solution adjusted to 7% by mass, 360 g of phenol, 2.4 g of dodecanethiol, and 12 g of PC/PET alloy pellets (PC content 68% by mass: 1.2 g × 68% by mass ÷ 100 ÷ 254 g/mol = 0.003 mol) were placed at room temperature in a nitrogen atmosphere. The resulting reaction liquid was in the form of a slurry.
Then, the internal temperature was increased to 90°C, and the reaction liquid was subjected to reaction for 4 hours while the internal temperature was maintained at 90°C to obtain a homogeneous decomposed liquid.
This decomposed liquid was filtered through a glass filter equipped with a vacuum pump so that 586 g of a filtrate was obtained as a homogeneous solution R. Part of this homogeneous solution R was subjected to composition analysis by high-performance liquid chromatography. As a result, generation of bisphenol A was confirmed.

(Step 1)

The obtained homogeneous solution R was gradually cooled from 90°C to 10°C to obtain a slurry. The obtained slurry was filtered so that 179 g of a crude cake R (solid component) and 373 g of a crude crystallization mother liquor were obtained.

(Step 2)

450 g of demineralized water heated to 60°C was sprinkled on the crude cake R to wash the crude cake so that 141 g of a purified cake R was obtained. Part of this purified cake R was subjected to composition analysis by high-performance liquid chromatography and, as a result, it was confirmed that the purified cake R was composed of a crystalline adduct that was a phenol adduct of bisphenol A.

(Adsorption Crystallization Step)

The obtained purified cake R was returned into the jacket-type separable flask equipped with a Dimroth condenser, a stirring blade, and a thermometer, 600 g of toluene was added thereto, and the resultant was heated to 75°C to melt the purified cake R so that an organic phase R1 containing bisphenol A (bisphenol solution) was obtained.
This organic phase R1 was subjected to suction filtration by supplying it little by little to a glass filter loaded with 20 g of activated carbon to obtain an organic phase R2 (adsorption-purified liquid). Filterability was excellent.

(Water Washing Step)

The obtained organic phase R2 was again returned into the separable flask, 100 g of water was added thereto, the resultant was stirred for 15 minutes while being maintained at 75°C, and the resultant was then allowed to stand for 10 minutes to separate it into two phases so that an organic phase and a water phase were obtained. This operation was repeated three times to obtain an organic phase R3. The water phase obtained by the fourth operation was discharged into

a heatproof jar and cooled to 25°C, and then the electric conductivity thereof was measured and found to be 2.1 μS/cm.

(Purifying Crystallization Step)

Then, the organic phase R3 was cooled to 10°C to obtain a slurry. The obtained slurry was filtered so that 78 g of a cake was obtained.

The obtained cake was dried using a rotary evaporator while being maintained at 85°C for 5 hours so that 63 g of bisphenol A was obtained.

The yield was 58 mol% (63 g ÷ 228 g/mol ÷ 0.475 mol × 100 = 58 mol%). The obtained bisphenol A was analyzed by gas chromatography and, as a result, the content of toluene was 500 ppm by mass. It should be noted that as a result of analysis of the bisphenol A after drying to determine the amount of polymers contained therein, the amount of a component having a molecular weight of 10000 or more was equal to or less than a detection lower limit (0.1% by mass or less).

[Example 13]

(Depolymerization Step)

In a jacket-type separable flask equipped with a Dimroth condenser, a stirring blade, and a thermometer, 120 g of a polycarbonate resin (120 g ÷ 254 g/mol = 0.472 mol, Molecular weight of repeating unit of polycarbonate resin: 254 g/mol), 107 g of an aqueous sodium hydrogen carbonate solution adjusted to 7% by mass, 360 g of phenol, 2.4 g of dodecanethiol, and 12 g of PC/PBT alloy pellets (PC content 67% by mass: 1.2 g × 67% by mass ÷ 100 ÷ 254 g/mol = 0.003 mol) were placed at room temperature in a nitrogen atmosphere. The resulting reaction liquid was in the form of a slurry.

Then, the internal temperature was increased to 90°C, and the reaction liquid was subjected to reaction for 4 hours while the internal temperature was maintained at 90°C to obtain a decomposed liquid. The decomposed liquid was in a suspended form.

This decomposed liquid was filtered through a glass filter equipped with a vacuum pump so that 572 g of a filtrate was obtained as a homogeneous solution S. Part of this homogeneous solution S was subjected to composition analysis by high-performance liquid chromatography. As a result, generation of bisphenol A was confirmed.

(Step 1)

The obtained homogeneous solution S was gradually cooled from 90°C to 10°C to obtain a slurry. The obtained slurry was filtered 30 so that 175 g of a crude cake S (solid component) and 368 g of a crude crystallization mother liquor were obtained.

(Step 2)

450 g of demineralized water heated to 60°C was sprinkled on the crude cake S to wash the crude cake so that 136 g of a purified cake S was obtained. Part of this purified cake S was subjected to composition analysis by high-performance liquid chromatography and, as a result, it was confirmed that the purified cake S was composed of a crystalline adduct that was a phenol adduct of bisphenol A.

(Adsorption Crystallization Step)

The obtained purified cake S was returned into the jacket-type separable flask equipped with a Dimroth condenser, a stirring blade, and a thermometer, 600 g of toluene was added thereto, and the resultant was heated to 75°C to melt the purified cake S so that an organic phase S1 containing bisphenol A (bisphenol solution) was obtained.

This organic phase S1 was subjected to suction filtration by supplying it little by little to a glass filter loaded with 20 g of activated carbon to obtain an organic phase S2 (adsorption-purified liquid). Filterability was excellent.

(Water Washing Step)

The obtained organic phase S2 was again returned into the separable flask, 100 g of water was added thereto, the resultant was stirred for 15 minutes while being maintained at 75°C, and the resultant was then allowed to stand for 10 minutes to separate it into two phases so that an organic phase and a water phase were obtained. This operation was repeated three times to obtain an organic phase S3. The water phase obtained by the fourth operation was discharged into a heatproof jar and cooled to 25°C, and then the electric conductivity thereof was measured and found to be 1.8 μS/cm.

(Purifying Crystallization Step)

Then, the organic phase S3 was cooled to 10°C to obtain a slurry. The obtained slurry was filtered so that 73 g of a cake was obtained.
The obtained cake was dried using a rotary evaporator while being maintained at 85°C for 5 hours so that 60 g of bisphenol A was obtained.
The yield was 55 mol% (60 g ÷ 228 g/mol ÷ 0.475 mol × 100 = 55 mol%). The obtained bisphenol A was analyzed by gas chromatography and, as a result, the content of toluene was 500 ppm by mass. It should be noted that as a result of analysis of the bisphenol A after drying to determine the amount of polymers contained therein, the amount of a component having a molecular weight of 10000 or more was equal to or less than a detection lower limit (0.1% by mass or less).

[Table 3]

| | Type of polymer | Homogeneous solution | Product BPA | |
|---|---|---|---|---|
| | | PHL/BPA (mol/mol) | Polymer content | TOL content |
| Example 11 | PC/ABS | 7.5 | 0.1 wt%≥ | 500 wtppm |
| Example 12 | PC/PET | 7.5 | 0.1 wt%≥ | 500 wtppm |
| Example 13 | PC/PBT | 7.5 | 0.1 wt%≥ | 500 wtppm |

[Example 14]

In a glass reaction vessel having an internal capacity of 150 mL and equipped with a stirrer and a distillate outlet tube, 60g of bisphenol A obtained in Example 9, 45 g of bisphenol A manufactured by Mitsubishi Chemical Corporation (0.46 mol as bisphenol A), 107.4 g of diphenyl carbonate (0.50 mol), and 7 μL of a 400 ppm by mass aqueous cesium carbonate solution were placed. The pressure in the glass reaction vessel was reduced to about 100 Pa and was then returned to atmospheric pressure by nitrogen. This operation was repeated three times to purge the inside of the reaction vessel with nitrogen. Then, the reaction vessel was immersed in an oil bath at 220°C to melt the contents.
The rotation speed of the stirrer was set to 100 rpm, and the pressure in the reaction vessel was reduced from an absolute pressure of 101.3 kPa to 13.3 kPa in 40 minutes while phenol produced as a by-product of an oligomerization reaction between bisphenol A and diphenyl carbonate in the reaction vessel was distilled off.
Then, a transesterification reaction was performed for 80 minutes while the pressure in the reaction vessel was maintained at 13.3 kPa and phenol was further distilled off.
Then, the external temperature of the reaction vessel was increased to 290°C and the pressure in the reaction vessel was reduced from an absolute pressure of 13.3 kPa to 399 Pa in 40 minutes so that phenol was discharged as a distillate to the outside of the system.
Then, the pressure in the reaction vessel was reduced to an absolute pressure of 30 Pa and a polycondensation reaction was performed. The polycondensation reaction was terminated at the time when the stirring power of stirrer of the reaction vessel reached a predetermined level. The time from when the external temperature of the reaction vessel reached 290°C until when the polymerization was terminated was 133 minutes.
Then, the pressure in the reaction vessel was returned to an absolute pressure of 101.3 kPa by nitrogen and was then increased to a gauge pressure of 0.2 MPa, and a polycarbonate resin was taken out of the reaction vessel to obtain the polycarbonate resin. The obtained polycarbonate resin had a viscosity-average molecular weight (Mv) of 20170 and a pellet YI of 12.9.

[Example 15]

As a polycarbonate resin complex, a polycarbonate resin composition coated with an acrylic cured product was used. The polycarbonate resin composition coated with an acrylic cured product was prepared by forming a 60 mm × 60 mm × 3 mm plate using a polycarbonate resin "NOVAREX (registered trademark) M7022J" manufactured by Mitsubishi Chemical Engineering Plastics Co., Ltd., applying "ACRYKING (registered trademark)" manufactured by Mitsubishi Chemical Corporation onto the plate and drying and then curing it by UV irradiation, and cutting the plate into pieces of about 5 to 10 mm.

(Depolymerization Step)

In a jacket-type separable flask equipped with a Dimroth condenser, a stirring blade, and a thermometer, 199 g of

the polycarbonate resin composition coated with an acrylic cured product (199 g ÷ 254 g/mol = 0.783 mol, Molecular weight of repeating unit of polycarbonate resin: 254 g/mol), 88.1 g of an aqueous sodium hydrogen carbonate solution adjusted to 7% by mass, 299 g of phenol, and 2.0 g of dodecanethiol were placed at room temperature in a nitrogen atmosphere. The resulting reaction liquid was in the form of a slurry.

Then, the internal temperature was increased to 90°C, and the reaction liquid was subjected to reaction for 4 hours while the internal temperature was maintained at 90°C to obtain a decomposed liquid. The decomposed liquid was cloudy and was not a homogeneous solution.

This decomposed liquid was filtered through a glass filter equipped with a vacuum pump so that 553 g of a filtrate was obtained as a homogeneous solution T. Part of this homogeneous solution T was subjected to composition analysis by high-performance liquid chromatography. As a result, generation of bisphenol A was confirmed.

(Step 1)

The obtained homogeneous solution T was gradually cooled from 90°C to 10°C to obtain a slurry. The obtained slurry was filtered so that 226.7 g of a crude cake T (solid component) and 313.4 g of a crude crystallization mother liquor were obtained.

(Step 2)

400 g of demineralized water heated to 60°C was sprinkled on the crude cake T to wash the crude cake so that 201.5 g of a purified cake T was obtained. Part of this purified cake T was subjected to composition analysis by high-performance liquid chromatography and, as a result, it was confirmed that the purified cake T was composed of a crystalline adduct that was a phenol adduct of bisphenol A.

(Adsorption Purification Step)

The obtained purified cake T was returned into the jacket-type separable flask equipped with a Dimroth condenser, a stirring blade, and a thermometer, 600 g of toluene was added thereto, and the resultant was heated to 75°C to melt the purified cake T so that an organic phase T1 containing bisphenol A (bisphenol solution) was obtained.

This organic phase T1 was subjected to suction filtration by supplying it little by little to a glass filter loaded with 40 g of activated carbon to obtain an organic phase T2 (adsorption-purified liquid). Filterability was excellent.

(Water Washing Step)

The obtained organic phase T2 was again returned into the separable flask, 100 g of water was added thereto, the resultant was stirred for 15 minutes while being maintained at 75°C, and the resultant was then allowed to stand for 10 minutes to separate it into two phases so that an organic phase and a water phase were obtained. This operation was repeated three times to obtain an organic phase T3. The water phase obtained by the fourth operation was discharged into a heatproof jar and cooled to 25°C, and then the electric conductivity thereof was measured and found to be 2.1 μS/cm.

(Purifying Crystallization Step)

Then, the organic phase T3 was cooled to 10°C to obtain a slurry. The obtained slurry was filtered to obtain a cake, 300 g of toluene was further supplied to the cake to suspend and wash the cake, and the resultant was then filtered so that 117 g of a cake was obtained.

The obtained cake was dried using a rotary evaporator while being maintained at 85°C for 5 hours so that 101 g of bisphenol A was obtained.

The yield was 57 mol% (101 g ÷ 228 g/mol ÷ 0.783 mol × 100 = 57 mol%). The obtained bisphenol A was analyzed by gas chromatography and, as a result, the content of toluene was 400 ppm by mass. It should be noted that as a result of analysis of the bisphenol A after drying (product BPA) to determine the amount of polymers contained therein, the amount of a component having a molecular weight of 10000 or more was equal to or less than a detection lower limit (0.1% by mass or less).

[Example 16]

As a polycarbonate resin complex, a polycarbonate resin composition containing organic microparticles was used. The polycarbonate resin composition containing organic microparticles was prepared by adding 0.5% by mass of spherical organic microparticles (GM-0205S manufactured by Aica Kogyo Company, Limited) to a polycarbonate resin

"NOVAREX (registered trademark) M7022J" manufactured by Mitsubishi Chemical Engineering Plastics Co., Ltd., mixing the resultant in a tumbler for 20 minutes, supplying the resultant to "TEX30HSST" equipped with a single vent and manufactured by The Japan Steel Works, Ltd. to knead it under conditions of a screw rotation speed of 200 rpm, a discharge rate of 15 kg/hour, and a barrel temperature of 280°C, rapidly cooling a molten resin extruded in a strand shape in a water bath, and pelletizing the strand with a pelletizer.

(Depolymerization Step)

In a jacket-type separable flask equipped with a Dimroth condenser, a stirring blade, and a thermometer, 199 g of the polycarbonate resin composition containing organic microparticles (199 g ÷ 254 g/mol = 0.783 mol, Molecular weight of repeating unit of polycarbonate resin: 254 g/mol), 88.1 g of an aqueous sodium hydrogen carbonate solution adjusted to 7% by mass, and 299 g of phenol were placed at room temperature in a nitrogen atmosphere. The resulting reaction liquid was in the form of a slurry.

Then, the internal temperature was increased to 90°C, and the reaction liquid was subjected to reaction for 4 hours while the internal temperature was maintained at 90°C to obtain a homogenous decomposed liquid.

This decomposed liquid was filtered through a glass filter equipped with a vacuum pump so that 548 g of a filtrate was obtained as a homogeneous solution U. Part of this homogeneous solution U was subjected to composition analysis by high-performance liquid chromatography. As a result, generation of bisphenol A was confirmed.

(Step 1)

The obtained homogeneous solution U was gradually cooled from 90°C to 10°C to obtain a slurry. The obtained slurry was filtered so that 277 g of a crude cake U (solid component) and 272 g of a crude crystallization mother liquor were obtained.

(Step 2)

450 g of demineralized water heated to 60°C was sprinkled on the crude cake U to wash the crude cake so that 236 g of a purified cake U was obtained. Part of this purified cake U was subjected to composition analysis by high-performance liquid chromatography and, as a result, it was confirmed that the purified cake U was composed of a crystalline adduct that was a phenol adduct of bisphenol A.

(Adsorption Crystallization Step)

The obtained purified cake U was returned into the jacket-type separable flask equipped with a Dimroth condenser, a stirring blade, and a thermometer, 600 g of toluene was added thereto, and the resultant was heated to 75°C to melt the purified cake U so that an organic phase U1 containing bisphenol A (bisphenol solution) was obtained.

This organic phase U1 was subjected to suction filtration by supplying it little by little to a glass filter loaded with 40 g of activated carbon to obtain an organic phase U2 (adsorption-purified liquid). Filterability was excellent.

(Water Washing Step)

The obtained organic phase U2 was again returned into the separable flask, 100 g of water was added thereto, the resultant was stirred for 15 minutes while being maintained at 75°C, and the resultant was then allowed to stand for 10 minutes to separate it into two phases so that an organic phase and a water phase were obtained. This operation was repeated three times to obtain an organic phase U3. The water phase obtained by the fourth operation was discharged into a heatproof jar and cooled to 25°C, and then the electric conductivity thereof was measured and found to be 2.2 μS/cm.

(Purifying Crystallization Step)

Then, the organic phase U3 was cooled to 10°C to obtain a slurry. The obtained slurry was filtered to obtain a cake, 300 g of toluene was further supplied to the cake to suspend and wash the cake, and the resultant was filtered so that 141 g of a cake was obtained.

The obtained cake was dried using a rotary evaporator while being maintained at 85°C for 5 hours so that 120 g of bisphenol A was obtained.

The yield was 67 mol% (120 g ÷ 228 g/mol ÷ 0.783 mol × 100 = 67 mol%). The obtained bisphenol A was analyzed by gas chromatography and, as a result, the content of toluene was 500 ppm by mass. It should be noted that as a result of analysis of the bisphenol A after drying to determine the amount of polymers contained therein, the amount of a

component having a molecular weight of 10000 or more was equal to or less than a detection lower limit (0.1% by mass or less).

## Claims

1. A method for producing a bisphenol by removing, from a mixed solution containing a bisphenol and a resin other than a polycarbonate resin (hereinafter referred to as "another resin"), the another resin to obtain the bisphenol, the method comprising
a step 1 in which the bisphenol is precipitated out of an aromatic monoalcohol-containing solution in which the bisphenol and the another resin are dissolved in a solvent containing an aromatic monoalcohol to obtain a slurry of the bisphenol, and the slurry of the bisphenol is then subjected to solid-liquid separation to obtain a crude cake of the bisphenol and a mother liquor.

2. The method for producing a bisphenol according to claim 1, wherein before the bisphenol is precipitated, the mixed solution or the aromatic monoalcohol-containing solution is subjected to solid-liquid separation to obtain a homogeneous solution.

3. The method for producing a bisphenol according to claim 1, comprising, before the step 1, a depolymerization step in which a polycarbonate resin contained in a polycarbonate resin complex containing the polycarbonate resin and the another resin is subjected to depolymerization to obtain a decomposed liquid containing the bisphenol and the another resin, wherein
the mixed solution corresponds to the decomposed liquid.

4. The method for producing a bisphenol according to claim 3, wherein

   the mixed solution is one obtained by performing the depolymerization in the presence of the aromatic monoalcohol in the depolymerization step, and
   the aromatic monoalcohol-containing solution corresponds to the mixed solution.

5. The method for producing a bisphenol according to claim 3, wherein

   the mixed solution is one obtained by performing the depolymerization in the absence of the aromatic monoalcohol in the depolymerization step, and
   the aromatic monoalcohol-containing solution is one obtained by replacing a solvent of the obtained mixed solution with the aromatic monoalcohol.

6. The method for producing a bisphenol according to any one of claims 3 to 5, wherein the polycarbonate resin complex is at least one selected from the group consisting of the following (c1) to (c8):

   (c1) a molded body of a polymer alloy of the polycarbonate resin and the another resin;
   (c2) a resin molded body of the polycarbonate resin, the resin molded body having a surface coated with the another resin;
   (c3) a resin molded body of the polycarbonate resin, the resin molded body having a surface coated with a polymer alloy of the polycarbonate resin and the another resin;
   (c4) a resin molded body of a polymer alloy of the polycarbonate resin and the another resin, the resin molded body having a surface coated with the another resin;
   (c5) a resin molded body of a polymer alloy of the polycarbonate resin and the another resin, the resin molded body having a surface coated with a polymer alloy of the polycarbonate resin and the another resin;
   (c6) a resin molded body of a polymer alloy of the polycarbonate resin and the another resin, the resin molded body having a surface coated with the polycarbonate resin;
   (c7) a resin molded body of the another resin, the resin molded body having a surface coated with the polycarbonate resin; and
   (c8) a resin molded body of the another resin, the resin molded body having a surface coated with a polymer alloy of the polycarbonate resin and the another resin.

7. The method for producing a bisphenol according to any one of claims 1 to 3, wherein the aromatic monoalcohol contains phenol or cresol.

**8.** The method for producing a bisphenol according to any one of claims 1 to 3, wherein

the aromatic monoalcohol is phenol, and
in the step 1, the bisphenol is precipitated as a crystalline adduct of the bisphenol and phenol to obtain a crude cake of the crystalline adduct of the bisphenol and phenol.

**9.** The method for producing a bisphenol according to any one of claims 1 to 3, wherein an amount-of-substance ratio of the aromatic monoalcohol to the bisphenol contained in the aromatic monoalcohol-containing solution, out of which the bisphenol is to be precipitated, is 1.0 or more.

**10.** The method for producing a bisphenol according to any one of claims 1 to 3, comprising, after the step 1, a step 2 in which a washing liquid is supplied to the crude cake of the bisphenol to wash the crude cake of the bisphenol, so that a purified cake of the bisphenol is obtained.

**11.** The method for producing a bisphenol according to claim 10, wherein the washing liquid is at least one selected from the group consisting of an aromatic monoalcohol, an aliphatic monoalcohol, a ketone, an aromatic hydrocarbon, an aliphatic hydrocarbon, and water.

**12.** The method for producing a bisphenol according to any one of claims 1 to 3, comprising a step in which the crude cake of the bisphenol or a purified cake obtained by washing the crude cake is dissolved in a solvent or melted to obtain a bisphenol solution, and the bisphenol solution is then brought into contact with a solid adsorbent to obtain an adsorption-purified liquid.

**13.** The method for producing a bisphenol according to any one of claims 1 to 3, wherein the another resin is at least one selected from the group consisting of an acrylic resin, polyethylene terephthalate, polybutylene terephthalate, an ABS resin, a polyamide, a phenol resin, a polyurethane, polylactic acid, and a silicone resin.

**14.** The method for producing a bisphenol according to any one of claims 1 to 3, wherein the bisphenol is 2,2-bis(4-hydroxyphenyl)propane.

**15.** A method for producing a recycled polycarbonate resin, the method comprising the steps of:

obtaining a bisphenol by the method for producing a bisphenol according to any one of claims 1 to 3; and
producing a recycled polycarbonate resin by using a bisphenol raw material containing the obtained bisphenol.

Fig. 1

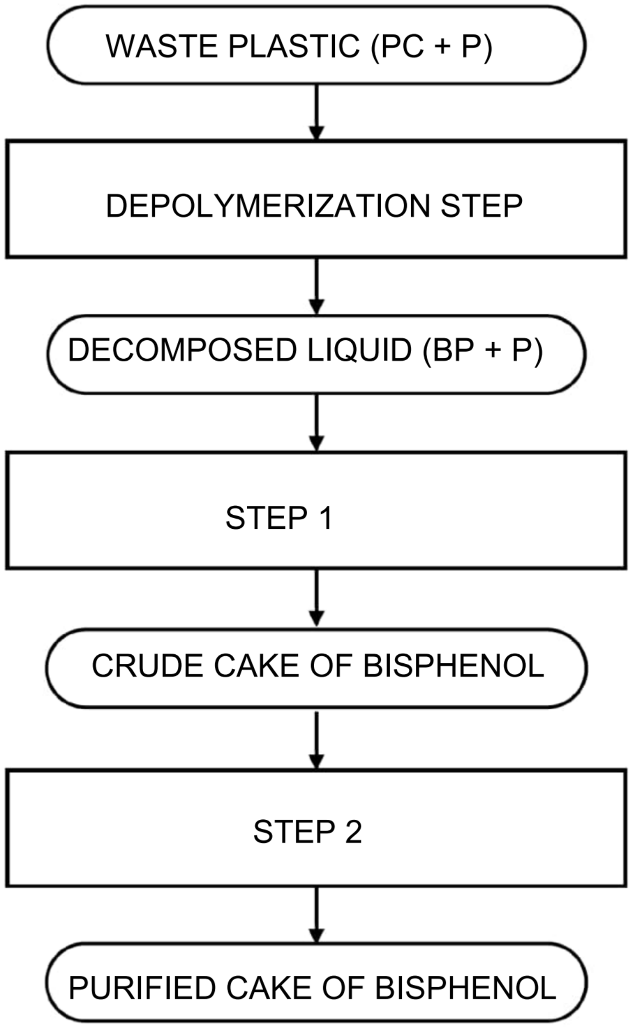
WASTE PLASTIC (PC + P)
DEPOLYMERIZATION STEP
DECOMPOSED LIQUID (BP + P)
STEP 1
CRUDE CAKE OF BISPHENOL
STEP 2
PURIFIED CAKE OF BISPHENOL

Fig. 2

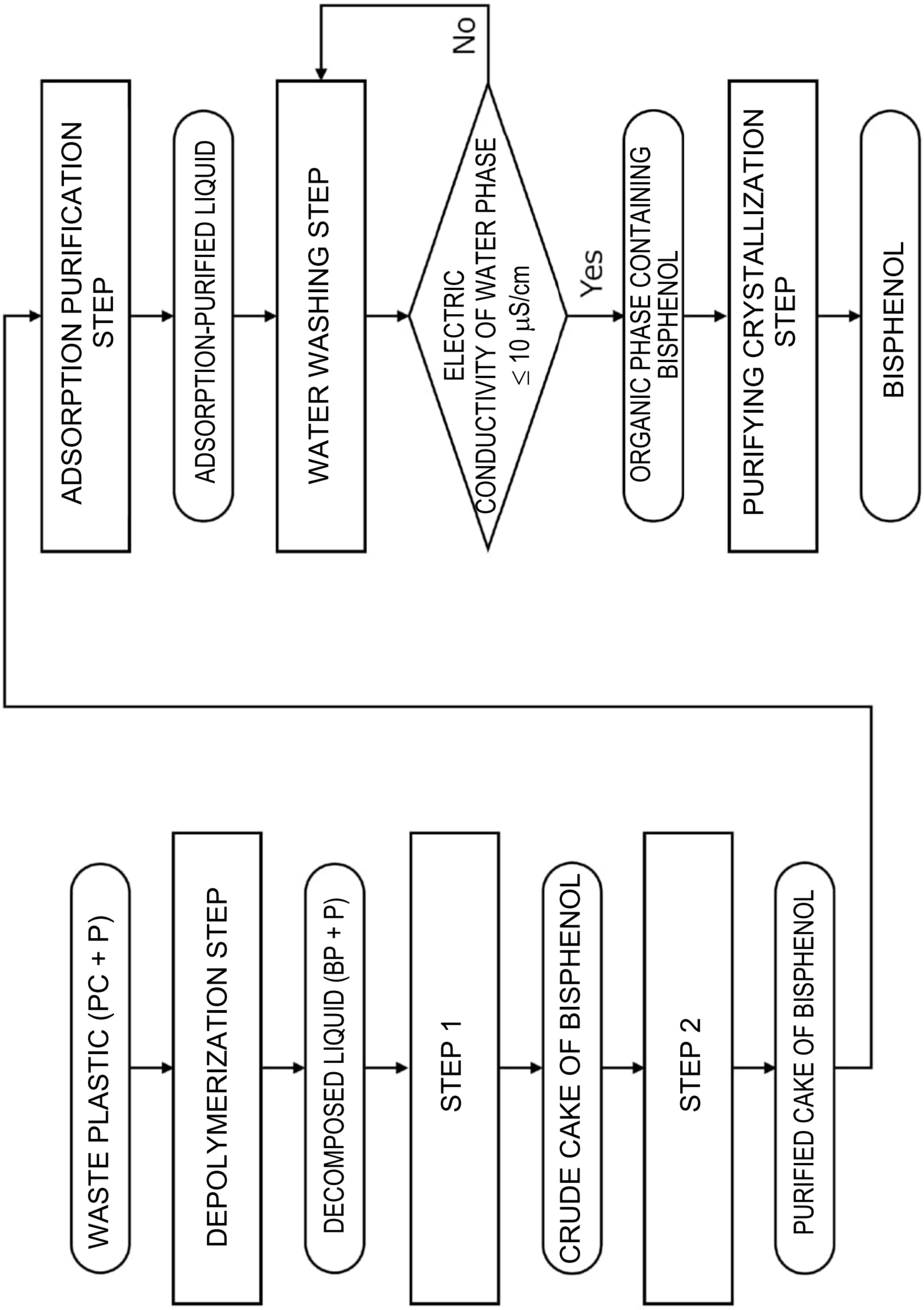
WASTE PLASTIC (PC + P)
DEPOLYMERIZATION STEP
DECOMPOSED LIQUID (BP + P)
STEP 1
CRUDE CAKE OF BISPHENOL
STEP 2
PURIFIED CAKE OF BISPHENOL
ADSORPTION PURIFICATION STEP
ADSORPTION-PURIFIED LIQUID
WATER WASHING STEP
ELECTRIC CONDUCTIVITY OF WATER PHASE ≤ 10 μS/cm
No
Yes
ORGANIC PHASE CONTAINING BISPHENOL
PURIFYING CRYSTALLIZATION STEP
BISPHENOL

Fig. 3

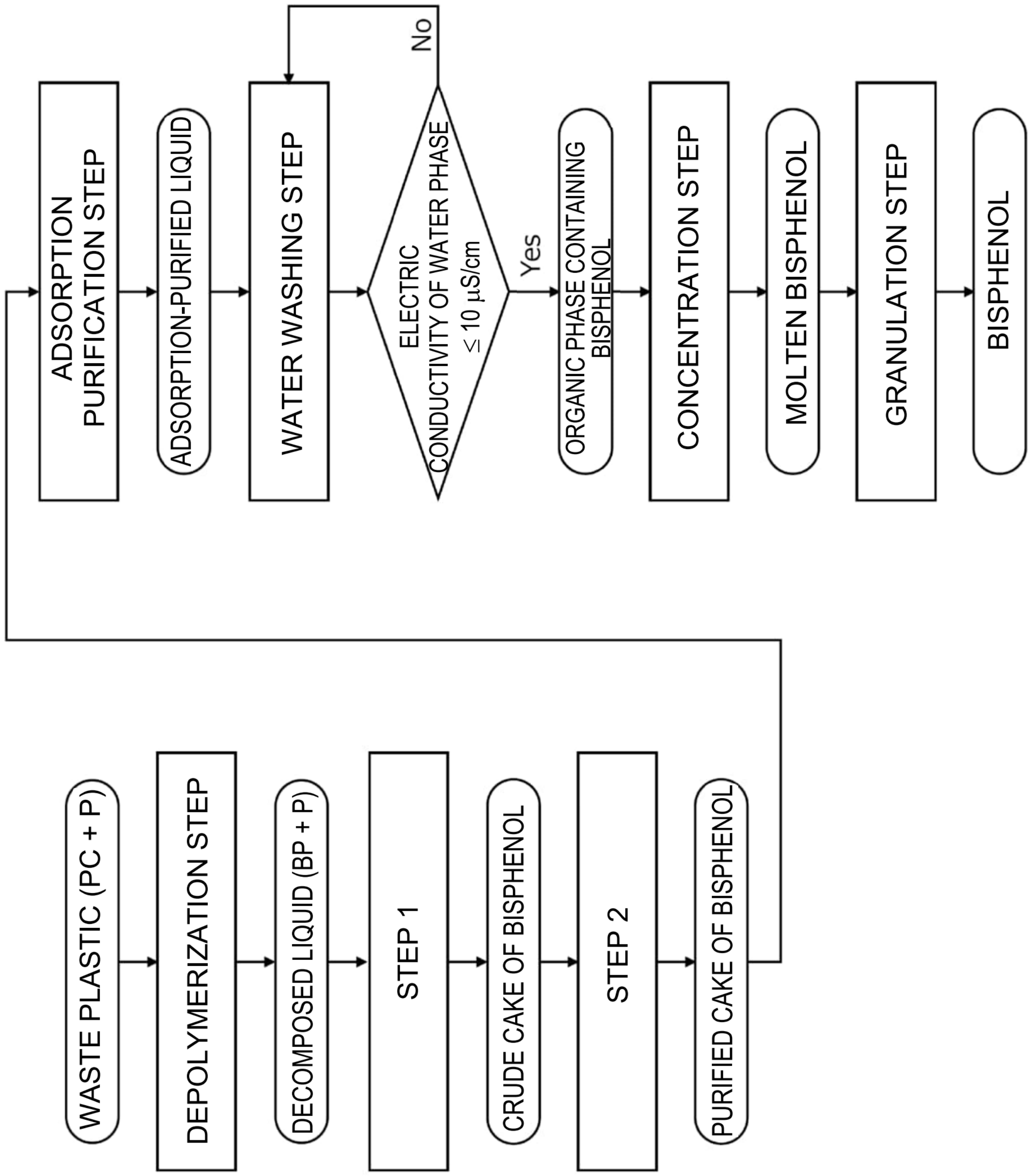
WASTE PLASTIC (PC + P)
DEPOLYMERIZATION STEP
DECOMPOSED LIQUID (BP + P)
STEP 1
CRUDE CAKE OF BISPHENOL
STEP 2
PURIFIED CAKE OF BISPHENOL
ADSORPTION PURIFICATION STEP
ADSORPTION-PURIFIED LIQUID
WATER WASHING STEP
ELECTRIC CONDUCTIVITY OF WATER PHASE ≤ 10 μS/cm
No
Yes
ORGANIC PHASE CONTAINING BISPHENOL
CONCENTRATION STEP
MOLTEN BISPHENOL
GRANULATION STEP
BISPHENOL

Fig. 4

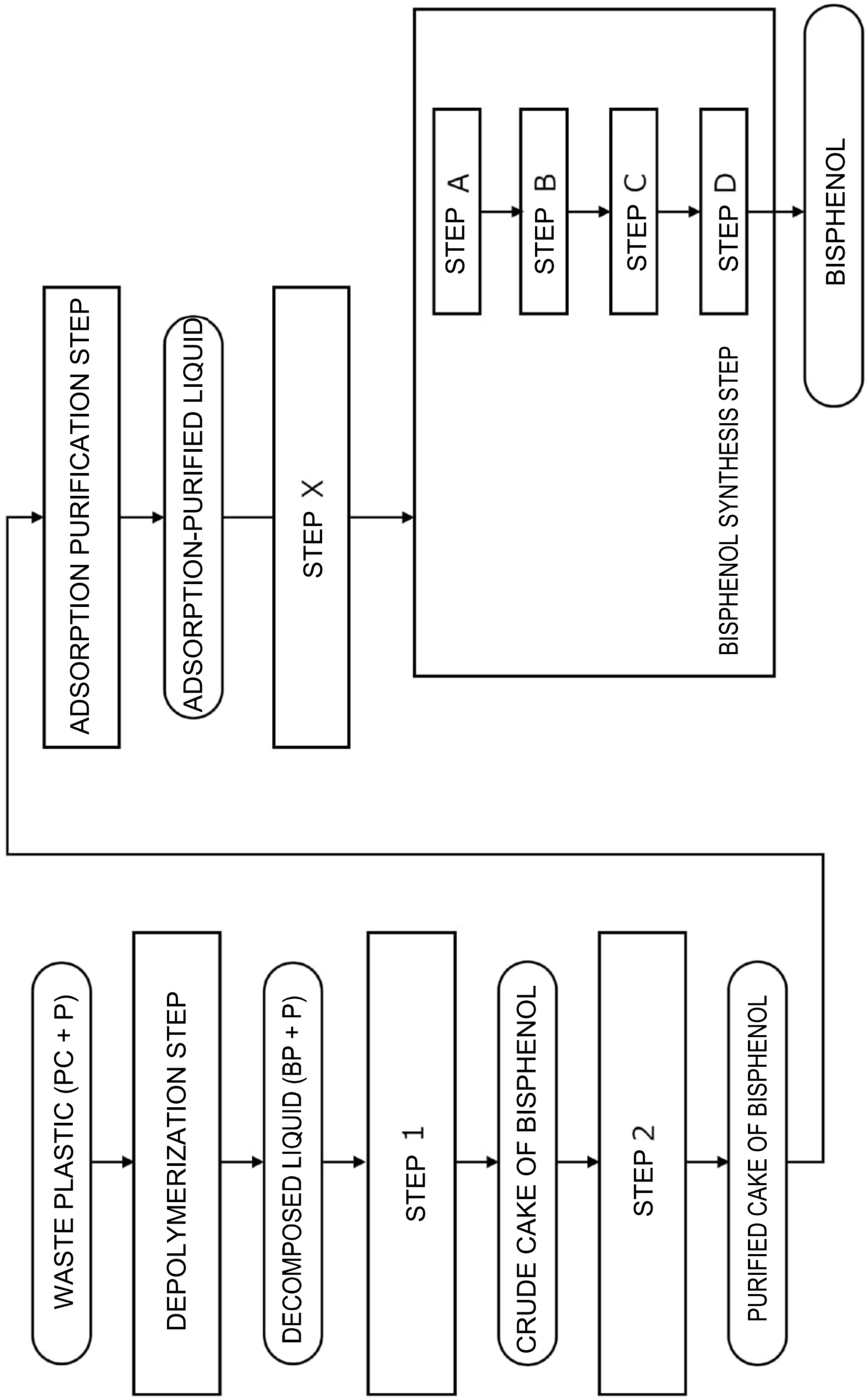
WASTE PLASTIC (PC + P)
DEPOLYMERIZATION STEP
DECOMPOSED LIQUID (BP + P)
STEP 1
CRUDE CAKE OF BISPHENOL
STEP 2
PURIFIED CAKE OF BISPHENOL
ADSORPTION PURIFICATION STEP
ADSORPTION-PURIFIED LIQUID
STEP X
STEP A
STEP B
STEP C
STEP D
BISPHENOL SYNTHESIS STEP
BISPHENOL

Fig. 5

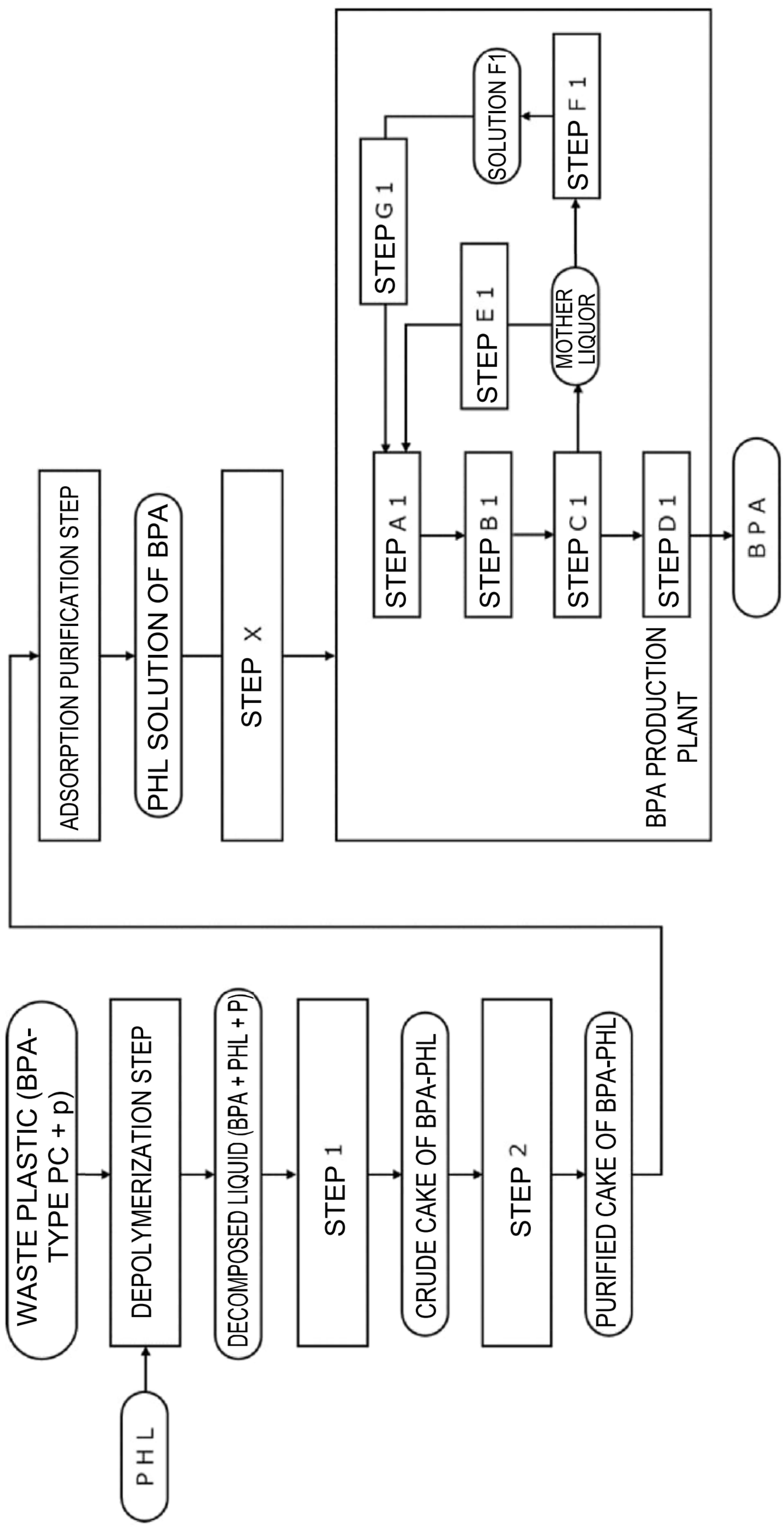
WASTE PLASTIC (BPA-TYPE PC + p)
PHL
DEPOLYMERIZATION STEP
DECOMPOSED LIQUID (BPA + PHL + P)
STEP 1
CRUDE CAKE OF BPA-PHL
STEP 2
PURIFIED CAKE OF BPA-PHL
ADSORPTION PURIFICATION STEP
PHL SOLUTION OF BPA
STEP X
BPA PRODUCTION PLANT
STEP A 1
STEP B 1
STEP C 1
STEP D 1
BPA
MOTHER LIQUOR
STEP E 1
STEP F 1
SOLUTION F1
STEP G 1

## INTERNATIONAL SEARCH REPORT

International application No.
**PCT/JP2024/001424**

**A. CLASSIFICATION OF SUBJECT MATTER**

***C07C 37/84***(2006.01)i; ***C07C 37/52***(2006.01)i; ***C07C 39/16***(2006.01)i; ***C08G 64/20***(2006.01)i
FI: C07C37/84; C07C37/52; C07C39/16; C08G64/20

According to International Patent Classification (IPC) or to both national classification and IPC

**B. FIELDS SEARCHED**

Minimum documentation searched (classification system followed by classification symbols)

C07C37/84; C07C37/52; C07C39/16; C08G64/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); JSTChina (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2005-112781 A (VICTOR CO. OF JAPAN LTD.) 28 April 2005 (2005-04-28)<br>entire text, all drawings | 1-15 |
| A | JP 2006-36668 A (TSUKISHIMA KIKAI CO., LTD.) 09 February 2006 (2006-02-09)<br>entire text, all drawings | 1-15 |
| A | JP 2005-179267 A (TEIJIN CHEM. LTD.) 07 July 2005 (2005-07-07)<br>entire text, all drawings | 1-15 |
| A | JP 2023-5691 A (MITSUBISHI CHEMICAL CORPORATION) 18 January 2023 (2023-01-18)<br>entire text, all drawings | 1-15 |
| A | JP 2022-103564 A (MITSUBISHI CHEMICAL CORPORATION) 08 July 2022 (2022-07-08)<br>entire text, all drawings | 1-15 |
| A | JP 2019-99525 A (MITSUBISHI CHEMICAL CORPORATION) 24 June 2019 (2019-06-24)<br>entire text, all drawings | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 March 2024** | **02 April 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/001424**

**C. DOCUMENTS CONSIDERED TO BE RELEVANT**

| Category* | Citation of document, with indication, where appropriate, of the relevant passages | Relevant to claim No. |
|---|---|---|
| P, A | JP 2023-13730 A (MITSUBISHI CHEMICAL CORPORATION) 26 January 2023 (2023-01-26)<br>entire text, all drawings | 1-15 |

Form PCT/ISA/210 (second sheet) (July 2022)

**INTERNATIONAL SEARCH REPORT**
**Information on patent family members**

International application No.
**PCT/JP2024/001424**

| Patent document cited in search report | Publication date (day/month/year) | Patent family member(s) | Publication date (day/month/year) |
|---|---|---|---|
| JP 2005-112781 A | 28 April 2005 | (Family: none) | |
| JP 2006-36668 A | 09 February 2006 | (Family: none) | |
| JP 2005-179267 A | 07 July 2005 | (Family: none) | |
| JP 2023-5691 A | 18 January 2023 | (Family: none) | |
| JP 2022-103564 A | 08 July 2022 | (Family: none) | |
| JP 2019-99525 A | 24 June 2019 | (Family: none) | |
| JP 2023-13730 A | 26 January 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2001310970 A
- JP 2009084538 A
- JP 2005179460 A
- JP 2001160243 A
- JP 7207059 A

### Non-patent literature cited in the description

- **MITSUBISHI**. NOVAREX (registered trademark) M7027BF. *Chemical Engineering Plastics*
- **MITSUBISHI**. MB2105. *Chemical Engineering*
- **MITSUBISHI**. MB4309R. *Chemical Engineering*
- **MITSUBISHI**. MB2213R. *Chemical Engineering*